# NEW EUROPEAN PATENT SPECIFICATION

(11) **EP 2 254 622 B2**
(45) Date of publication and mention of the opposition decision: **02.12.2026**
(45) Mention of the grant of the patent: 01.05.2019
(21) Application number: 09707113.8
(22) Date of filing: 06.02.2009
(51) Int. Cl.: A61M 39/26, A61M 5/158, A61M 5/142, A61M 5/14

(54) **INSERTER ASSEMBLY**
EINFÜHRUNGSANORDNUNG
ENSEMBLE D'INSERTION

(30) Priority: 08.02.2008 DK 200800185; 08.02.2008 US 27125 P; 13.02.2008 DK 200800202; 13.02.2008 US 28259 P; 13.02.2008 DK 200800203; 13.02.2008 US 28262 P; 20.02.2008 US 30022 P; 21.02.2008 DK 200800240; 25.02.2008 DK 200800262; 25.02.2008 US 31227 P; 07.08.2008 US 187971; 09.09.2008 US 95379 P
(43) Date of publication of application: 01.12.2010
(60) Divisional application: 12154607.1 / 2 452 709; 21213225.2 / 3 988 147
(73) Proprietor: Unomedical A/S, 3460 Birkerød (DK)
(72) Inventor: HØRDUM, Elo, Lau, DK-2970 Hørsholm (DK); GYRN, Steffen, DK-4100 Ringsted (DK); HICKMOTT, Richard, Morgan, DK-2100 København Ø (DK); MORTON, Alistair, David, DK-2770 Kastrup (DK); HASTED, Søren, Bo, DK-4180 Sorø (DK); JEPPESEN, Henrik, DK-2840 Holte (DK)
(74) Representative: D Young & Co LLP
(86) International application number: PCT/EP2009/051399
(87) International publication number: WO 2009/098306

(56) References cited:
- EP-A1- 2 259 816
- WO-A1-2004/030726
- WO-A1-2007/071255
- WO-A1-2009/001345
- WO-A1-2009/001346
- WO-A1-2009/001347
- WO-A2-2004/098684
- WO-A2-2005/018703
- WO-A2-2006/061027
- US-A- 5 267 963
- US-A1- 2002 077 599
- US-A1- 2004 158 207
- US-A1- 2006 253 085
- US-A1- 2007 282 269

## Description

### Technical field of the invention

The invention concerns an assembly comprising an inserter device comprising an insertion needle for inserting a medical device in the form of a penetrating member into the subcutaneous or intramuscular area of a patient, a medical device to be placed subcutaneously or intramuscularly attached to the inserter device before use and a base part to be attached to the skin of a patient wherein the medical device is attached during use.

### Background of the invention

US 2004/0158207 relates to a device for inserting a cannula into tissue, including a cannula a protective element which can accommodate said cannula, an operating element for moving the cannula out of the protective element, and a holder fixedly connected to the cannula. The invention encompasses a system for connecting liquid supply to the cannula after insertion i.e. this assembly comprises a base part, a penetrating member and an inserter. The contact surface between the assembly and the patients skin just before insertion is the same as the contact surface between the assembly and the patients skin after insertion of the penetrating member and removal of the inserter. Other examples of this type of devices according to the preamble of claim 1 are known from WO 2005/018703 and WO 2004/030726.

The assembly comprising the three elements is sold as a unit in a sterile packing. When the user is to utilize the assembly, the assembly is removed from the sterile packing, and then the assembly is positioned on a surface of the patients' skin, the surface of the assembly being in contact with the patients skin being a surface of the base part. When the assembly has been properly attached, the inserter device is actuated by which the medical device is released from the inserter device and attached to the base part in a position where a part of the medical device is placed subcutaneously or intramus-cularly. After placement of the medical device the inserter device can be removed from the assembly leaving the medical device combined with the base part on the patients skin.

The assembly of the present invention can be constructed with a relatively low profile.

Thus, there is an obvious need in the art for a robust, reliable, accurate, safe, hygienic, and user friendly insertions device, which addresses the issues discussed above.

### Summary of the invention

The current invention provides an assembly comprising an insertion device for subcutaneously introduction of a penetrating member, where a "penetrating member" is understood to be a needle, a cannula, a sensor or the like. The penetrating member is normally prior and during insertion kept in a position where it is not visible to the patient and where it can not get in contact with the user or the patient before it is actually inserted.

The object of the invention is to provide an assembly comprising an inserter device (10), a penetrating member (7) and a base part (100), where
- the base part (100) comprises a surface adapted to be attached to a skin surface, a position adapted to receive and/or attach to the penetrating member (7), and means (14) adapted to secure the base part to the inserter device (10),
- the penetrating member (7) comprises a part to be placed subcutaneously or intramuscularly, a body (24) which is in contact with the inserter device (10) during insertion and with the base part (100) during use, and
- the inserter device (10) comprises a cavity for receiving the penetrating member (7), means (45) for accelerating the penetrating member (7) and bringing the penetrating member (7) to the receiving position in the base part (100) and means for penetrating the skin of the patient,
   wherein the length of the joined assembly (lₜₒₜₐₗ) before use is larger than the length of the base part (100) (l₂) alone.

"Before use" is considered to be before insertion and e.g. also before the assembly is removed from a sterile packing.

According to an embodiment of the assembly the height of the joined assembly (hₜₒₜₐₗ) before use is smaller than the individual heights of the inserter device (10) (h₁) and the base part (10) (h₂) added together.

"Height" of a device in this connection is considered to be the longest dimension which can be measured from a surface closest to the patients skin to a surface most distant from the patients skin in a direction perpendicular to the patients skin.

"Length" of a device in this connection is considered to be the longest dimension which can be measured from one end to another in a direction horizontal to the patients skin. Basically, the inserter and the base part are placed at least partially beside each other in stead of on top of each other before and during insertion. This result in a relatively stable assembly as it has a relatively large contact surface to the patients skin, and normally the inserter device will have a removal direction which is different - and not just opposite - from the insertion direction. That the inserter device is detached and removed from the base part in a direction different from the insertion direction makes it less likely that the penetrating member is pulled away from the in-use-position during removal of the inserter device.

The inserter device comprises
- a moving part (38) comprising guiding means (39) which guiding means (39) restrict the movement of the penetrating member (50) and guide the penetrating member (50) from a first to a second position in a first direction, i.e. the direction of insertion, towards the injection site, and
- a stationary housing (30) comprising guiding means (32) which guiding means (32) restrict the movement of the moving part (38), and
- the penetrating member (50) comprises transformation means (52) corresponding to the guiding means (39) of the moving part (38).

According to an embodiment of the assembly the guiding means (32) guide the moving part (38) in a second direction which is linear and different from the first direction i.e. the direction of insertion.

According to an embodiment of the assembly the base part and the penetrating member comprises corresponding means adapted to attach the penetrating member (50) to the base part (100) during use and after insertion.

According to an embodiment of the assembly the inserter (10) comprises inserter attachment means (14) locking the inserter (10) to the base part before and during insertion of the penetrating member (7) which inserter attachment means (14) can be unlocked so that the inserted can be removed from the base part after insertion of the penetrating member (7), the inserter attachment means (14, 14A, 14B, 14PR/R, 14PL/L) comprise at least one protruding part and at least one corresponding opening.

According to an embodiment of the assembly either the at least one protruding part or the at least one corresponding opening part is positioned on a surface of the base part and a corresponding part either comprising a protruding part or an opening is positioned on a surface of the inserter (10).

According to an embodiment of the assembly the inserter (10) is released from the base part by applying a force to the inserter (10) or a part of the inserter (10) in a direction different from the direction of insertion of the penetrating member (7).

According to an embodiment of the assembly the release of the inserter (10) from the base part is at least partly provided by the release of a force in direction towards the base part.

According to an embodiment of the assembly the released force is provided by a spring unit (45) exercising a force directed to a proximal surface of the inserter housing and a distal surface of the base part (1).

According to an embodiment of the assembly the spring (36) is a leaf spring which at one end is fastened unreleasably to a part of the inserter and at the other end will touch the upper surface of the base part before the inserter is released from the base part.

According to an embodiment of the assembly the penetrating part (50) which comprises a cannula part (7) together with the base part (100) which base part (100) is provided with an internal fluid path and constitutes an infusion part provided with a cannula part during use, and
- the cannula part (7) comprises a body (24) formed by a hard material having an inner through going opening which through going opening is in fluid contact with a cannula (22) providing fluid contact with the patient, the body (24) of the cannula part (7) has an opening (20) corresponding to the inlet or outlet opening (12) of the internal fluid path resulting in fluid contact between the internal fluid path and the cannula part (7) and these two corresponding openings (12, 20) do, when they are positioned opposite each other, allow unrestricted flow
- the internal fluid path comprises at least one inlet and one outlet opening (12, 13) through which a fluid can enter and exit the fluid path, and
- a sealing (18) is positioned between the cannula part (7) and the inlet/outlet opening (12) of the fluid path when the cannula part (7) is in position for use in order to keep the fluid path to the cannula tight.

According to an embodiment of the assembly the sealing (18) is surrounding the inlet/outlet opening (12) *and*/*or* the distance d₁ between a centre line c of the cannula part and a point on the outer surface of the cannula part positioned at or above the upper edge of the sealing (18) is larger than the distance d₂ between the centre line c of the cannula part and a point on the outer surface of the cannula part positioned at or below the lower edge of the sealing (18).

According to an embodiment of the assembly the body (24) of the cannula part (7) is provided with a sealing (18) before use or the opening (12) of the fluid path is provided with a sealing (18) before use.

According to an embodiment of the assembly the body (24) of the cannula part (7) has at least a second opening (21) to the inner through going opening.

According to an embodiment of the assembly the second opening (21) to the inner through going opening is covered by a self closing membrane which membrane can be penetrated by a blunt or pointy needle.

According to an embodiment of the assembly a membrane (17) completely covers an opening (13) giving access to a space in the assembly which membrane (17) is made of an elastic material penetrable by a needle, the membrane (17) is fastened around the opening (13) and the membrane (17) protrudes from the opening (13) and forms an air filled volume in front of the opening (13) which air filled volume can be reduced in size when a pressure is put on the membrane (17) from the outside, the inner surfaces of the membrane (17) define a passage (17a) at the first closed end of the membrane (17) through which a needle (19) can pass and the second end of the membrane (17) is adapted to attach the membrane (17) to a holding part (61).

According to an embodiment of the assembly the walls of the membrane (17) have a thickness and shape of the chosen membrane material making it possible, to maintain the protruding shape in a use position without the outer surfaces of the membrane (17) being supported with walls of rigid material.

According to an embodiment of the assembly the second end of the membrane (17) has an internal opening (17b) fitting around the outer contour of a protruding part (61a) on the holding part (61) and that the membrane (17) is fastened to the holding part (61) by interference fit.

According to an embodiment of the assembly the membrane (17) is supported with walls of a rigid material on the inner surfaces of the membrane (17)e.g. in the form of a penetrating needle.

According to an embodiment of the assembly the cross-section of the air-filled volume in the passage (17a) is smaller than 2 · [max cross-section of the opening (13)].

According to an embodiment of the assembly the assembly comprises a fluid connection (60) having at least a first and a second opening (13, 12), i.e. an inlet and an outlet, where the first opening (13) forms a fluid connection to a medication supply (6) or the like and the second opening (12) forms a fluid connection to an opening in the body (24) of a separate cannula part (7) and an at least partly sub- or transcutaneous positioned cannula (22).

According to an embodiment of the assembly the fluid connection is attached to a surface plate (1) and has the form of a tube (60) made of a rigid material; normally the fluid connection (60) is fastened to the surface plate (1) by a holding part (61). According to an embodiment of the assembly the tube (60) is made of metal or plastic e.g. the tube (60) comprises a hollow needle made e.g. of steel. The tube (60) normally has a diameter or maximum cross-section ≤ 1 mm.

According to an embodiment of the assembly the tube (60) has at least one pointy end (19) protruding from the holding part (61) and e.g. the tube (60) also has a blunt end.

According to an embodiment of the assembly the pointy end of the tube (60) forms a connector needle (19) being the inlet to a connector part (3) and when pushing a reservoir (6) towards the inlet the connector needle (19) penetrates a membrane (17) completely covering a first opening (13) of the connector part (3). The tube (60) might consists of a single piece.

According to an embodiment of the assembly the tube (60) is bend in an angle > 0 degrees in at least one position or the tube (60) is bend in an angle > 0 degrees in at least two positions.

### Definitions

"Parallel" or "essentially parallel" as used herein refers to a second movement in a direction, plane, item or the like defined in relation to a first or a reference plane or direction which reference plane or direction has a direction defined as the angle α = 0°; and the second plane or direction deviates at maximum ±10°; normally not more than ±5° from the first or reference direction α.

In the context of the application "horizontal" or "essentially horizontal" means that a movement in a direction, a direction, plane, item or the like is horizontal or essentially horizontal is parallel or essentially parallel to the surface of the skin of a patient as defined above. For example, the base part to which the insertion device is fastened can be horizontal, or essentially horizontal, parallel or essentially parallel to the skin.

"Perpendicular" or "essentially perpendicular" as used herein refers to a second movement in a direction, a direction, plane, item or the like defined in relation to a reference plane or direction which reference plane or direction has a position or a direction in the angle β = 0°; and the second plane or direction deviates between 80-100°; normally between 85-95° from the first reference β.

In the context of the application "Transversal" or "essentially transversal" can be used interchangeably with perpendicular or essentially perpendicular as defined above.

"Means": As used herein, the expression means can comprise one or more means. This is irrespective, if with respect to grammar, the verb relating to said means indicates singular or plural.

### Brief description of the drawings

A detailed description of embodiments of the current invention will be made with reference to the accompanying figures, wherein like numerals designate corresponding parts in different figures.
Figs. 1A-C show a cross section of a first embodiment of an insertion device according to the invention in three states: A: before activation; B: just after insertion; C: after retraction of insertion needle.
Figs. 2A-F show a cross section of a second embodiment of an insertion device in six states. A: before activation; B: after activation, C: just after insertion, D: after retraction of insertion needle, E: after release of inserter housing and F: after removal of insertion device from base part.
Fig. 3 shows a first embodiment of an assembly comprising an insertion device according to the invention.
Fig. 4 shows a second embodiment of an assembly comprising an insertion device according to the invention.
Figs. 5A, 5B, 5C and 5D show the second embodiment of the assembly, in fig. 5A the insertion device is mounted on the base part, and in fig. 5B and 5C the insertion device has been removed from the base part, in fig. 5D it is indicated how the reservoir is positioned when a delivery part is attached to the base part.
Figure 6 shows a second embodiment of the assembly without the insertion device and having the delivery part separated from the base part A: seen from below; B: seen from above; C: seen from above and showing the connection part of the base part; D: showing the base part alone seen from above; and E: shows a third embodiment of a base part to be used with the assembly.
Fig. 7 shows a longitudinal cut through an assembly as shown in fig. 3-6, the cut is placed at the position of one of the fastening means for the insertion device.
Fig. 8 shows the insertion device without being attached to the base part.
Fig. 9A and 9B show a third embodiment of an inserter to be used with the assembly respectively in a state before and after insertion of a cannula part.
Fig. 10A and 10B show an embodiment of a base part.
Figs. 11A-C show how the embodiment of the inserter shown in fig. 9 and 10 are being detached from a base part of an assembly according to the invention.
Figs. 12A and 12B show an embodiment of a moving part to be used with an assembly as shown in fig. 9-11.
Figs. 13A and 13B show a fourth embodiment of an inserter to be used with the assembly in a state after insertion of a cannula part.
Figs. 14A and 14B show the internal parts of the inserter housing of the fourth embodiment of the inserter.
Figs. 15A and 15B show an embodiment of a moving part to be used with an assembly as shown in fig. 13-14.
Fig. 16A shows one embodiment of a penetrating member which can be used with the assembly, fig. 16B and C shows a second embodiment of a penetrating member.
Fig. 17A and 17B shows a cut-through view of a second embodiment of a penetrating member which can be used with the assembly.
Fig. 18a shows an embodiment of a moving part having an increased tolerance. Fig. 18b shows how the moving part 38 is viewed in fig. 18a.
Fig. 19 shows a driving mechanism comprising a flat spring which can be used to drive the moving part forward in any of the illustrated embodiments of the inserter.
Fig. 20 shows an embodiment of a fluid path between reservoir and cannula part.
Fig. 21 shows a connector part which can be part of an infusion part according to the invention.
Fig. 22 shows the same connector part as fig. 21 without the bubble membrane covering the inlet.
Figs. 23A, 23B and 23C show a cannula part which can be used in connection with the invention.
Figs. 24A-24D show an enlargement of the contact between the cannula part and the cannula opening of the connection part.
Figs. 25A, B, C show an embodiment of a base part provided with a fluid path mainly constructed of a tube. Fig. 25D shows an alternative embodiment of the fluid path.
Fig. 26 shows an embodiment of an infusion part having an angle d = 90° between insertion direction and tangent to contact surface.
Fig. 27 shows a cannula part which can be used in connection with the invention.
Fig. 28 shows a detailed outline of the contact between a base part and a penetrating part.
Fig. 29 shows a cut-through view of a membrane to be used according to the invention.
Fig. 30 shows an enlargement of a second embodiment of a membrane to be used according to the invention
Fig. 31A-C shows several embodiments of sealings in the form of a bubble shaped membranes which can be used in connection with the invention.
Fig. 32 shows an embodiment of a fluid path in a base part.

### Detailed description of the invention

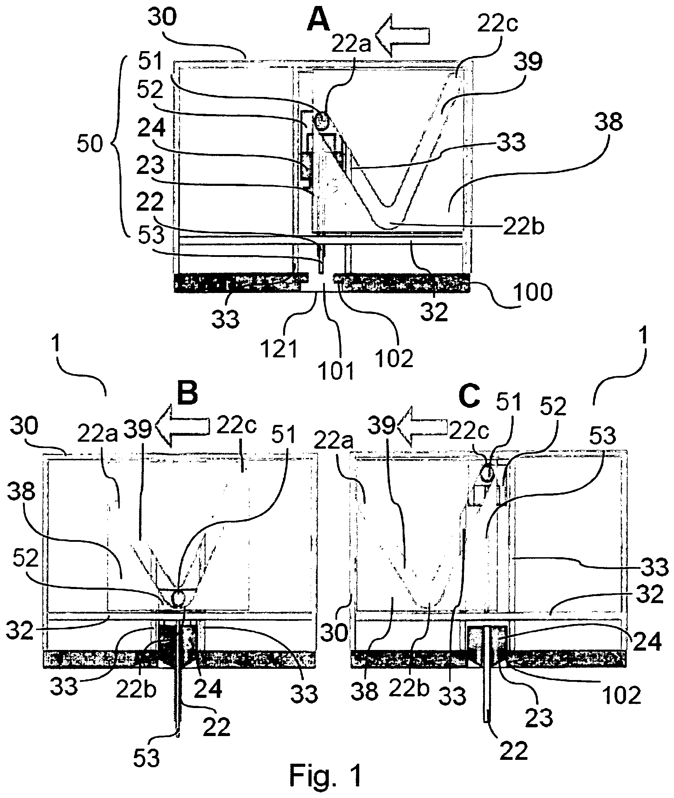

Figures 1A-1C shows one embodiment of an insertion device 1 for inserting a penetrating member 50 according to the present invention.

The insertion device 1 comprises a housing 30, a base part 100, a moving part 38 and a penetrating member 50. For clarity, the moving part 38 is represented in a semi-transparent fashion. The figs. 1A, 1B and 1C show the penetrating member 50 in three different positions relative to the moving part 38.

The penetrating member 50 comprises holding means 52 holding the penetrating member 50, transformation means 51 attached to the holding means 52 of the penetrating member 50, a body 24, a cannula 22, and an insertion needle 53. The cannula 22 is according to this embodiment a soft cannula which needs to be inserted with the help of an insertion needle 53 which is attached unreleasably to a part of the insertion device and not to the penetrating member 50. The cannula 22 is attached unreleasably to the body 24. Furthermore, the body 24 comprises retention means 23 for fastening of the cannula 22 to the base part 100 when the cannula 22 has been fully inserted. According to this embodiment the retention means 23 are formed as mechanical hooks which can be forced inward i.e. toward the centre where the cannula 22 is positioned. As the mechanical hooks are fastened to the body 24 in a flexible way the hooks will return to their original position after having been forced towards the centre, the flexibility will normally be due to the properties of the material used to produce the body, the hooks and the connection formed between them.

In another embodiment of the invention, the penetrating member 50 comprises a sensor or both a sensor and a cannula. In a further embodiment of the invention, the penetrating member 50 comprises more than one cannula 22 e.g. a plurality of cannula and/or a plurality of sensors.

The housing 30 comprises guiding means 32 for the moving part 38 and guiding means 33 for the penetrating member 50. The guiding means 32 for the moving part 38 according to this embodiment comprises surfaces of the inner walls of the housing 30 along which the moving part 38 can slide and the guiding means 33 for the penetrating member 50 comprises an upright tube-like shape. The moving part 38 is provided with transformation means in the form of a V-shaped opening which is form to fit closely with the transformation means 51 of the penetrating member 50. The housing 30 is releasably connected to the base part 100, and can be disconnected from the base part 100 after the penetrating member 50 has been inserted. When connected, the housing 30 and the base part 100 encloses the penetrating member 50, the moving part 38, and the guiding means 32, 33 for the moving part 38 and the penetrating member 50, respectively thereby providing a unit.

The base part 100 comprises an opening 101, which is dimensioned to allow passage or entering of the penetrating member 50 or at least a part of it, such as the cannula 22, the injection needle 53 and the retention means 23.

The base part 100 and the housing 30 are normally individual elements, which elements can be separated in a reversible or an irreversible fashion. According to the present embodiment the opening 101 comprises interaction means 102, adapted to interact with the retention means 23 of the body of the penetrating member 50. The opening 101 can be closed and/or protected by a seal 121 which seal 121 is either removable or can be penetrated by the penetrating member 50. The seal 121 can cover a large area of the base part 100 and if the base part 100 is partly constituted by a mounting pad with an adhesive surface the seal 121 can be a release layer protecting the adhesive surface before use.

The guiding means 32 for the moving part 38 provides a directional controlled movement of the moving part 38 essentially within the housing 30. In the depicted embodiment the moving part 38 can move essentially parallel, i.e. essentially horizontal relative to the base part 100, guided by the guiding means 32. Such a movement can be characterised as a sliding movement.

The movement performed by the moving part 38 is a longitudinal movement, i.e. a linear movement relative to the housing 30. The means used to initiate and maintain the movement of the moving part 38 can either be provided directly by the user i.e. the user pushes or pulls the moving part 38 or it can be provided by mechanical means such as a spring which only has to be activated by the user

The guiding means 33 for the penetrating member 50 which are a part of or connected to the moving part 38 provide a movement of the penetrating member 50 in a direction different from the direction of movement of the moving part 38. This feature has at least two advantages: 1. the user's actions when activating or pushing the moving part 38 is less likely to influence the actual insertion of the penetrating member 50, and 2. the insertion device can be constructed in a smaller and more compact manner.

According to the embodiment of fig. 1 the direction of movement of the penetrating member 50 is essentially perpendicularly to the direction of movement of the moving part 38. The guiding means 33 for the penetrating member can comprise one or more parts which together provides a well defined track or tube along or in which the penetrating member can slide e.g. the guiding means 33 may comprise a hollow, cylindrical element fastened to the housing 30, the penetrating member 50 can move inside the cylindrical element along the longitudinal axis of said cylindrical element, comparable to the movement of a piston in a cylinder. Such a movement can be described as a sliding movement as the contact between the inner surfaces of the cylindrical element and the outer surfaces of the penetrating member 50 provides the guiding. Alternatively, the guiding means 33 of the penetrating member 50 can comprise one or more bars, governing the direction of movement of the penetrating member 50. As seen in fig. 1, the guiding means 33 for the penetrating member 50 according to this embodiment extend from the inner ceiling of the housing to the base part 100. The guiding means 33 of the penetrating member 50 is not necessarily attached to the base part 100. The guiding means 33 normally e.g. rest against and/or touch and/or are connected with the base part 100. In the depicted embodiment, the guiding means 33 of the penetrating member 50 is connected to the housing 30 at the inside of the upper surface ("ceiling"), and at one or more side ("wall") of the housing 30.

The guiding means 39 or the transformation means of the moving part 38 for the transformation means 51 of the penetrating member 50 defines a track. This track extends from a starting point 22a to a middle point 22b and ends at an end point 22c. As seen in fig. 1, this track is V-shaped, or essentially V-shaped. In the depicted embodiment, the guiding means 39 of the moving part 38 are provided as a continuous grove or through going opening within the moving part 38. The middle point 22b is closer to the base part 100 than the starting point 22a, and also closer to the base part 100 than the end point 22c, also, the starting point 22a is closer to the base part 100 than the end point 22c.

It is not essential how the starting point 22a and the end point 22c varies relative to each other, i.e. it would be possible to have an embodiment where the end point 22c is closer to base part 100 than start point 22a or an embodiment where the starting point 22a and the end point 22c have the same distance to the base part 100. It should though be assured that the starting point 22a is placed in a distance from the base part which is far enough to keep the end of the cannula 22 and the end of a separate insertion needle 53 inside the housing 30 before insertion.

According to the invention and as illustrated in fig. 1A-1C, the insertion device 1 is adapted to provide:
(i) a first state (fig. 1A), where the penetrating member 50 is in the starting position 22a, it is fully retracted and does not protrude from the housing 30 of the insertion device 1, the moving part 38 is in a start position in the right side of the housing 30;
(ii) a second state (fig. 1B), where the penetrating member 50 is in the middle point 22b, the part(s) of the penetrating member 50 which are to be inserted, such as the cannula 22 and/or an insertion needle 53, fully protrude the housing 30 through the opening 101 in the base part 100, and the moving part 38 has been moved forward to a middle position relative to the housing 30. The stationary guiding means 33 of the penetrating member 50 prevent the penetrating member 50 from moving in the same direction as the moving part 38 and only allows a "vertical" movement of the penetrating member 50 i.e. vertical is here to be understood as being perpendicular to "horizontal"; and
(iii) a third position (fig. 1C), where the part(s) of the penetrating member 50 to be inserted still protrude the housing 30, but the transformation means 51 together with the holding means 52 and the insertion needle 53are at the end point 22c and the insertion needle has been retracted from the injection site. The moving part 38 has reached the end of its travel to the left in the stationary housing. In the second position (ii) and in the third position (iii), the body 24 of the penetrating member 50 is retained through interaction between the retention means 23 of the body 24 of the penetrating member 50 and the interacting means 102 of the base part 100.

As shown, the horizontally forward movement of the moving part 38 is transformed into an insertion movement of the penetrating member 50 followed by a retraction movement of one or more parts of the penetrating member 50. This is achieved by the interaction of the guiding means 39 of the moving part 38 with the transformation means 51 of the penetrating member 50.

In the first position (i), the transformation means 51 of the penetrating member 50 are at the starting point 22a of the track/guiding means 39. When the moving part 38 is moved horizontally guided by its guiding means 32, the penetrating member 50 is moved downwards, i.e. "vertically" towards the base part 100. The speed of the movement of the moving part 38 and the slope of the guiding means 39 define the speed of the movement of the penetrating member 50, thus the speed of insertion i.e. the steeper the slope of the guiding means 39 are, the shorter time will be used to guide the penetrating member 50 from the retracted start position to the inserted position.

In the second position (ii), the transformation means 51 of the penetrating member 50 have reached the middle point 22b of the guiding means 39. At this point the direction of the slope of the guiding means 39 changes from downwards, i.e. towards the base part 100, to upwards, i.e. away from the base part 100. Thus the orientation of the slope of the guiding means 39 defines the direction of movement of the penetrating member 50. Further the forward horizontal movement of the moving part 38 produces a retraction movement of the holding means 52 of the penetrating member 50 and the insertion needle 53. If the cannula 22 is a hard self penetrating cannula there will be no need of a separate insertion needle 53 and also there will be no need to perform the last retraction part of the movement i.e. the last line of the V in the track 39 could be left out and the middle point 22b would be identical to the end point 22c.

In the third position (iii), the transformation means 51 of the penetrating member 50 have reached the end point 22c of the guiding means 39, and the holding means 52 of penetrating member 50 and the insertion needle 53 are fully retracted.

As seen in fig. 1, the moving part 38 does not protrude the housing 30. The arrow above the figure indicates the direction of movement of the moving part 38.

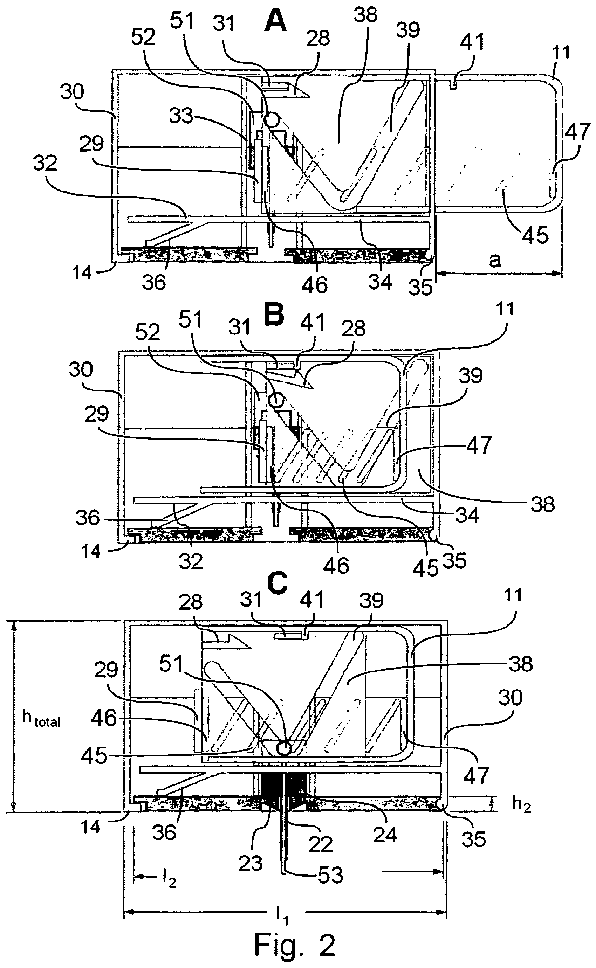

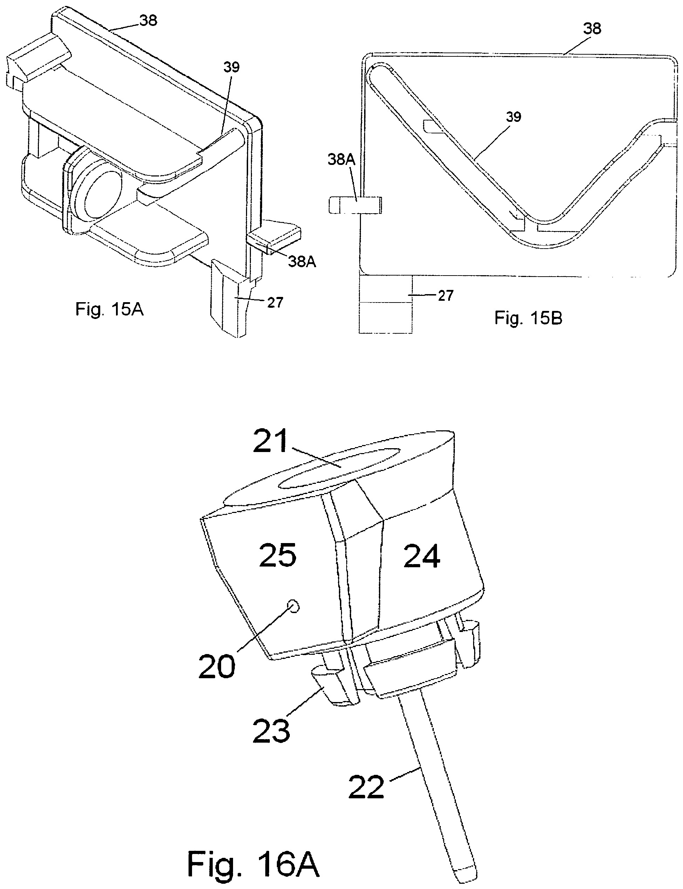

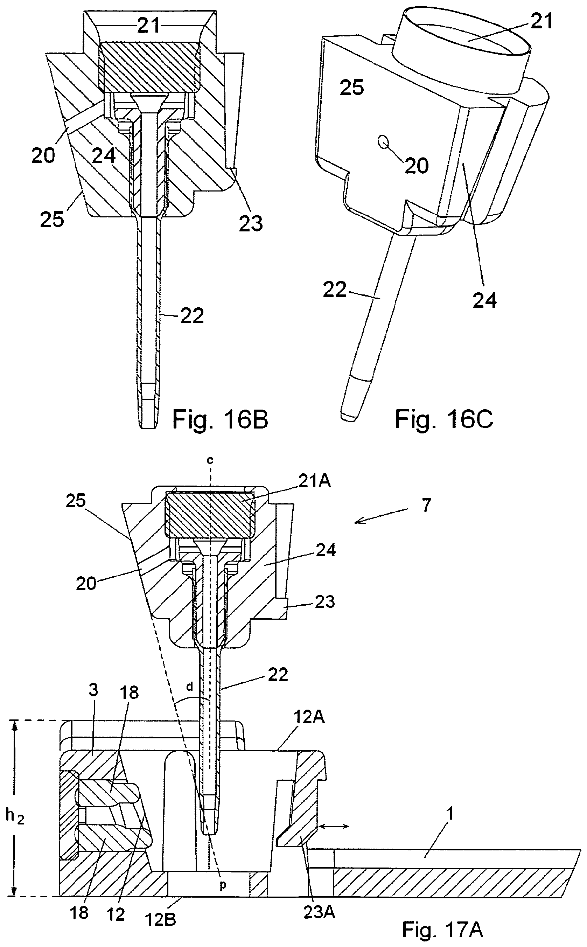

Fig. 2A-2F illustrates attachments means with an automatic release function. The insertion device 1 comprises a housing 30, a base part 100, a moving part 38, an activation part 11, and a penetrating member 50. One embodiment of a penetrating member 50 is shown in these figures but a penetrating member 50 similar to the penetrating members described in figure 16-17 might also be used. For illustrative purposes means the moving part 38 are represented in a semi-transparent fashion.

The housing 30 comprises guiding means 32 for the moving part 38 which allows the moving part 38 to move between at least two positions, guiding means 33 for the penetrating member 50 which allows the penetrating member 50 to move between at least two positions, and guiding means 34 for the activation part 11 which allows the activation part to move between at least two positions. The housing 30 is attached to the base part 100. According to this embodiment the attachment is releasable. The attachment is provided by parts of the housing 30 comprising a hinge 35 and fastening means 14 interacting with parts of the base part 100, whereby the housing 30 and the base part 100 are releasably connected. The hinge 35 comprises an at least partly rounded surface of a wall of the housing 30 which can pivot in relation to the base part 100 as it is placed in a groove in the base part 100. The fastening means 14 of the housing 30 interacts with locking means 108 of the base part 100.

The reference "hₜₒₜₐₗ" in fig. 2C indicates the total height of the housing 30 of the insertion device 1 and the base part. The height "hₜₒₜₐₗ" will expediently be in the range of 5-100 mm, and normally in the range 10-50 mm or more specifically 20-30 mm. The illustrated embodiment is 25 mm. In embodiments where the inserter is not removed after insertion of the penetrating part 50, the inserter should be as low as possible and normally not extend further from the patients skin than the delivery part 8. According to this embodiment the height h₁ of the housing is equal to the total height of the inserter 10 and the base part 100 i.e. hₜₒₜₐₗ < h₁ + h₂, and in this embodiment hₜₒₜₐₗ = h₁ as the inserter housing 30 fully comprise the base part 100 i.e. the two parts are not placed on top of each other in a way which make the complete assembly higher. The length of the joined assembly (lₜₒₜₐₗ) before use is larger than the length of the base part (100) (l₂) alone.

The housing 30 also comprises retention means 31. The retention means 31 hold the moving part 38 in a start position by engaging with locking means 28 on the moving part 38. According to this embodiment the retention means further provides a stop for the movement of the activation part 11.

The guiding means 32 for the moving part 38 provides a directional controlled movement of the moving part 38 in relation to the housing 30. The guiding means 32 are attached to or connected to or an integrated part of the inner surfaces of the housing 30 and will normally have the shape of longitudinal tracks corresponding to surfaces on the moving part 38 in order to make it possible for the moving part 38 to slide along the tracks. In the depicted embodiment, the moving part 38 can move parallel, i.e. horizontal to the base part 100, guided by the guiding means 32, the movement will normally be a sliding movement in a direction parallel to the surface of the base part 100, i.e. the movement is a longitudinal movement or a linear movement.

The guiding means 33 for the penetrating member 50 which are a part of or connected to or integrated with the housing 30 provides that the penetrating member 50 can only be moved in a well defined direction which direction is different from the direction of the moving member 38. In the embodiment the direction of movement of the penetrating member 50 is essentially perpendicularly to the direction of movement of the moving part 38. The guiding means 33 for the penetrating member 50 will normally be formed by inner surfaces of the housing 30, e.g. the guiding means 33 may comprise the inner surfaces of a hollow, cylindrical element wherein the penetrating member 50 can move between at least a forward and a retracted position along the longitudinal axis of said cylindrical element, comparable to the movement of a piston in a cylinder. If the penetrating member 50 has a rectangular cross-section the "cylindrical" element should of course be adapted to fit closely to the cross-section of the actually used penetrating member 50. Such a movement will be a sliding movement as the continuous contact between the inner surfaces of the cylindrical elements and the outer surfaces of the penetrating member 50 provides the guiding. Alternatively, the guiding means 33 of the penetrating member 50 can comprise one or more bars, governing the direction of movement of the penetrating member 50. As seen from the figures the guiding means 33 for the penetrating member 50 according to this embodiment can extend from the inner ceiling of the housing to the bottom part 100. The guiding means 33 of the penetrating member 50 are not attached to the base part 100 but might reach down and touch it or e.g. provide a support for the base part 100.

The guiding means 34 of the activation part 11 provides a directional controlled movement of the activation part 11 in relation to the housing 30. The guiding means 34 are attached to or integrated with the housing 30. In the depicted embodiment, the activation part 11 moves in parallel with, i.e. horizontal to the base part 100, guided by the guiding means 34 which according to this embodiment is provided as parts of the inner surfaces of the housing. The guiding means 34 might be formed as longitudinal tracks leading the activation part 11 in a well defined direction or simply the inner surfaces of the walls of the housing 30. Such a movement is normally a sliding movement as the guiding means 34 and the activation means are in continuous contact while moving in relation to each other. The movement will normally be a linear movement. The direction of movement of the activation part 11 is according to this embodiment identical to the direction of movement of the moving part 38 therefore the guiding means 34 of the activation part 11 can be the same as the guiding means 32 of the moving part 38 i.e. on set of guiding means 32, 34 provides the well defined and at least partly simultaneous movement of the moving part 38 and the activation part 11.

The moving part 38 is provided with transformation means 39 providing transformation of the movement of the moving part 38, which according to this embodiment is horizontal, into a movement of the penetrating member 50 in the insertion direction followed by a movement of at least the insertion needle of the penetrating member 50 in a direction of retraction. According to this embodiment the transformation means are in the form of a protruding cylindrical part 51 on the penetrating member 50 corresponding to an open V-shaped track 39 in the moving part 38. The V-shaped track 39 is sized to fit closely with the protruding part 51 of the penetrating member 50 in order to provide a well defined path of movement.

The moving part 38 comprises a releasing member 29 providing a separation of the housing 30, or at least a part of the housing 30, from the base part 100 by releasing the fastening means 14 of the housing from the locking means 108 of the base part 100. Said release is provided by interaction of the releasing member 29 with a part of the housing 30, according to this embodiment it is the inner wall of the housing 30 opposite the activation means 11 where the linear movement of the activation means 11 would end if continued to the inner wall of the housing 30.

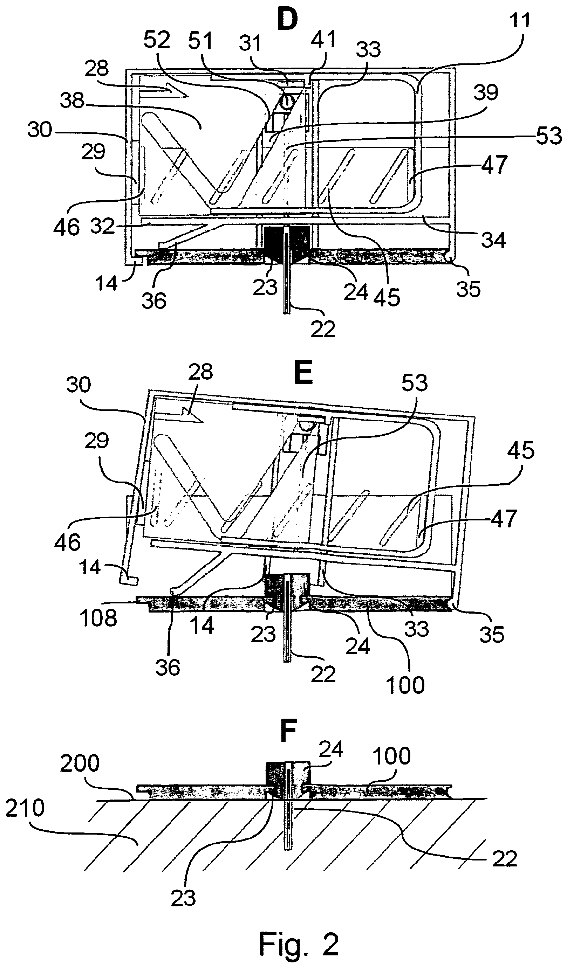

The housing comprises an elastic member 36 which, upon release of the fastening means 14 of the housing, initiates removal of the housing 30 from the base part 100. According to the embodiment shown in fig. 2 the elastic member 36 is an integrated part of the housing 30 i.e. it is fastened unreleasably to the housing 30. The elastic member 36 is a leaf spring unreleasably fastened to the housing 30 at one end and pressed against the base part 100 at the opposite end. The flexibility of the elastic member 36 is defined by the material of which it is constructed and the physical dimensions of the material, according to the present embodiment the elastic member is constructed of the same material as the housing i.e. a hard plastic and normally formed during molding of the housing 30, but it could also be constructed of a metal which after molding of the housing is fastened unreleasably to the housing 30.

Insertion of the penetrating member 50 using the insertion device according to the invention 1 is initiated by activation of the activation part 11. The activation part 11 is activated by pushing the part towards the housing 30. The activation part 11 comprises interaction means 41. The interaction means 41 interacts with the retention means 31 of the housing 30, thereby arresting the forward movement of the activation part 11. As can be seen in fig. 2A, the activation part 11 protrudes the housing 30 in the depicted, non-activated state. The letter "a" indicates the length of protrusion of the activation part 11 with respect to the housing 30. The protrusion before activation of the activation part 11 will normally be in the range of 1-100 mm, or 5-50 mm, or 10-25 mm, or 15-20 mm. In the shown embodiment the protrusion is 17 mm. In another embodiment of the invention, the activation part 11 does not protrude the housing 30, or protrudes the housing 30 only marginally.

The insertion device 1 is in a non-activated state before use, such as during transport or storage.

According to this embodiment a spring 45 is provided between the moving part 38 and the activation part 11. Normally the spring 45 will be in a relaxed state during storing as this will normally prolong the time the product can be stored while still being fully functional, if the spring 45 is in a biased state during storing there is a risk that the performance of the product will rapidly decrease. As illustrated in fig. 2A-F the spring 45 can be a spiral spring, comprising two ends: a first end 46, attached to, or placed in connection with the moving part 38 and a second end 47 attached to, or placed in connection with the activation part 11. The spring 45 is positioned along the direction of movement for the activation part 11 which is being parallel to the upper surface of the base part 100.

A function of the spring 45 is to provide energy for the penetration and/or retraction movement of the penetrating member 50 and/or parts of the penetrating member 50. If this energy is not provided by a spring 45 it has to be directly provided by the user of the device as the user provides a horizontal movement of the activation part 11 by pushing the activation part 11 towards the housing 30 and thereby a horizontal movement of the moving part 38.

The spring 45 of the illustrated embodiment stores energy from the movement of the actuation of the of the activation part 11 as the spring 45 is biased through this first movement. During actuation of the activation part 11 the moving part 38 is stationary. When the interaction means 41 of the activation part 11 gets into contact with the locking means 28, the moving part 38 is released from the stationary position and moved in a direction defined by the guiding means 32. The forward movement of the activation part 11 is stopped at the time where the interaction means 41 touches the retention means 31 of the housing 30. According to the embodiment of fig. 2 the direction of the moving part 38 is the same as the forward direction of the activation part 11. When the moving part 38 pushed by the spring 45 hits the inner surface of the housing 30, the spring 45 is biased enough to provide energy for the release of the releasable connection between the fastening means 14 of the housing 30 and the locking means 108 of the base part 100. This is provided by making the wall or at least a part of the wall of the housing 30 so flexible that the wall can be bend outward and release the fastening means 14 from the locking means 108 of the base part 100. When the locking connection is released the elastic member 36 pushes the housing 30 away from the base part 100 and the user will not need pull the insertion device away from the base part 100.

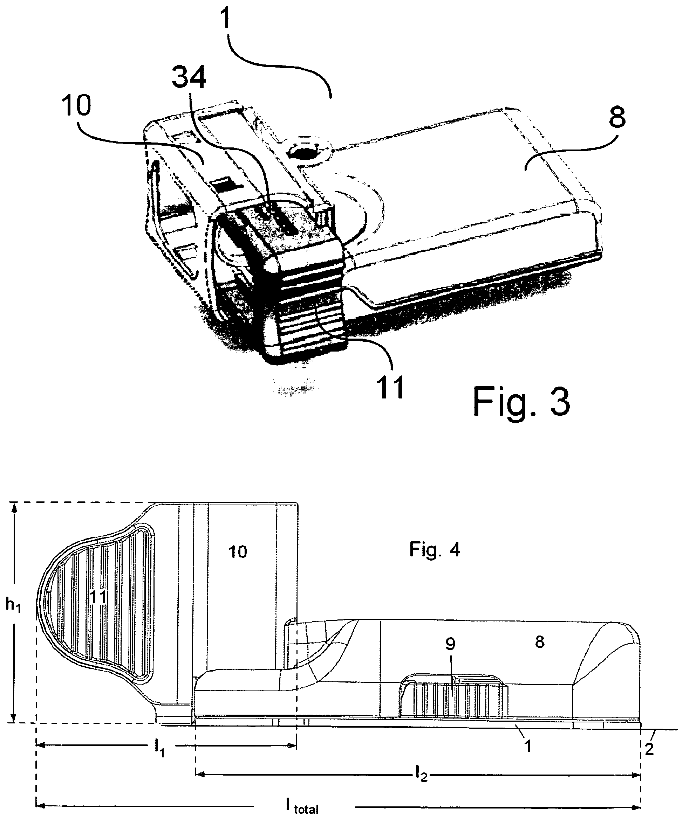

Figure 3 shows a first embodiment of an assembly comprising an inserter according to the invention together with a medication unit 8. Only the side of the base part 100 can be seen as the whole of the upper surface of the base part 100 is covered by the medication unit 8. The medication unit 8 will normally comprise both a reservoir for medication such as insulin and delivering parts in the form of pumping means and e.g. dosing means which can see to that the patient has a prescribed dose of medication.

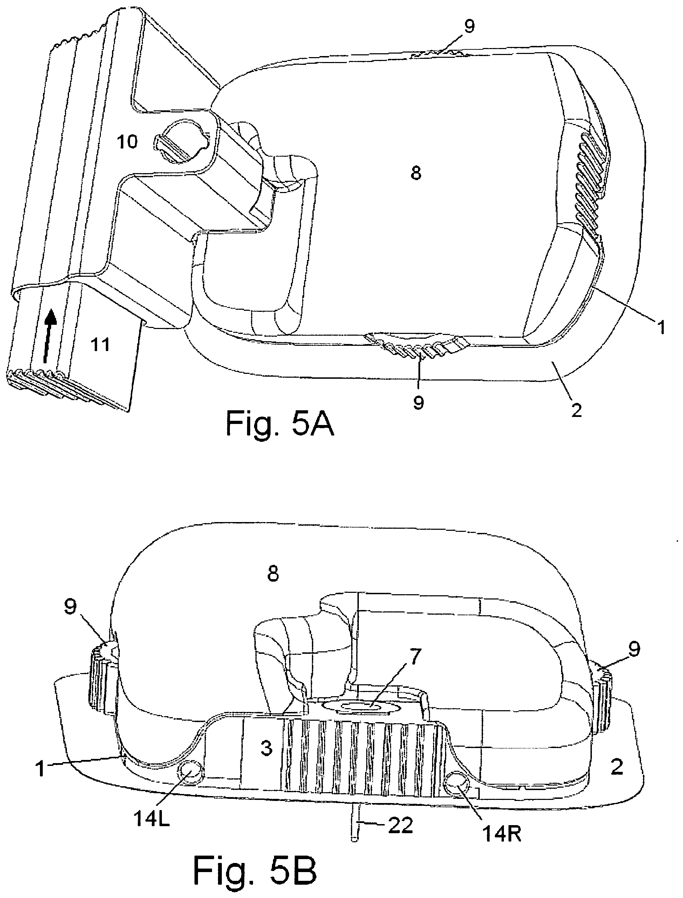

The figs. 4 and 5A and B show a second embodiment of an assembly comprising an inserter 10 according to the invention, a delivery part 8 and a base part. The base part comprises a surface plate 1 attached to a contact surface. The surface plate 1 is in this embodiment constructed of a molded plastic material and the contact surface is the proximal side of a mounting pad 2 which mounting pad 2 is unreleasably fastened to the surface plate 1 during manufacturing of the device. "Proximal" means the side or surface closest to the patient when the mounting pad is adhered to the patient, "distal" means the end or surface furthest away from the patient when the device is in a position of use.

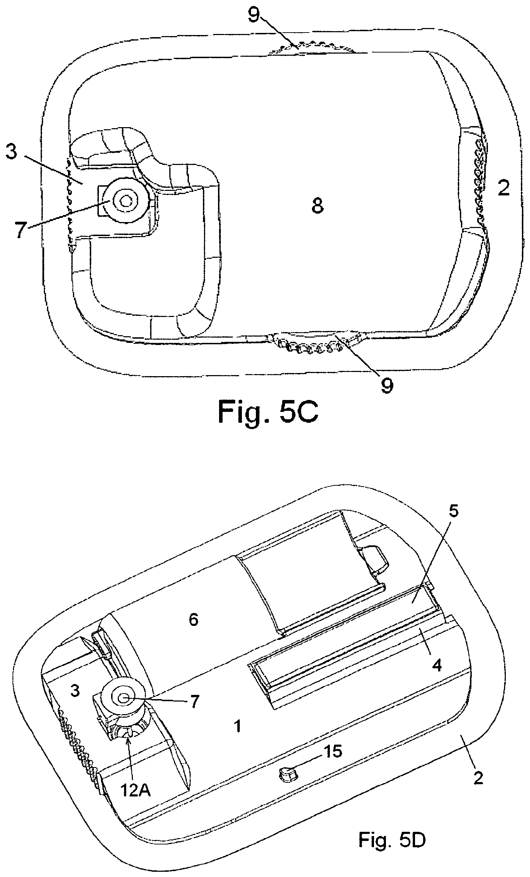

Fig. 4 shows the embodiment of the assembly seen from the side and fig. 5 shows the same embodiment seen from above.

In fig. 4 it the length of the different parts is illustrated. Also according to this embodiment the height h₁ of the inserter housing constitutes almost the total height of the inserter 10 and the base part 100 i.e. hₜₒₜₐₗ < h₁ + h₂, and in this embodiment hₜₒₜₐₗ ∼ h₁ as the inserter housing 30 comprises the connection part 3 of the base part 100 i.e. the two parts are not placed "end to end" although the base part 100 add a little to the total height. The length of the joined assembly lₜₒₜₐₗ before use i.e. before insertion and possible removal of the inserter housing, is larger than the sum of the length of the inserter housing l₁ and the length of the base part (100) l₂ i.e. lₜₒₜₐₗ < l₁ + l₂. Also l₂ < lₜₒₜₐₗ as it is the case for all the embodiments in the present document. The mounting pad 2 is not considered to contribute to the length or the height of the assembly.

The penetrating member of this embodiment is comprised in a cannula part 7 which is inserted into an opening 12A of a connector part 3 of the base part, this cannula opening 12A provides and opening which extends right through the base part. The cannula part 7 is provided with a penetrating member in the form of a cannula 22 which will penetrate the surface of the skin of the patient during the insertion and be positioned sub- or transcutaneously.

The inserter 10 holds the cannula part 7 before insertion and the insertion is initiated by pushing a handle 11. Fig. 5 shows the direction the handle 11 has to be pushed in order to initiate insertion of the cannula part 7. After insertion a not shown insertion needle can be retracted to the inside of the inserter 10, afterwards the inserter 10 can be removed from the base part, leaving an inserted cannula 22 fastened to the surface plate 1. If the cannula 22 of the cannula part 7 is a hard self penetrating cannula there will be no separate insertion needle and therefore no need to retract the insertion needle.

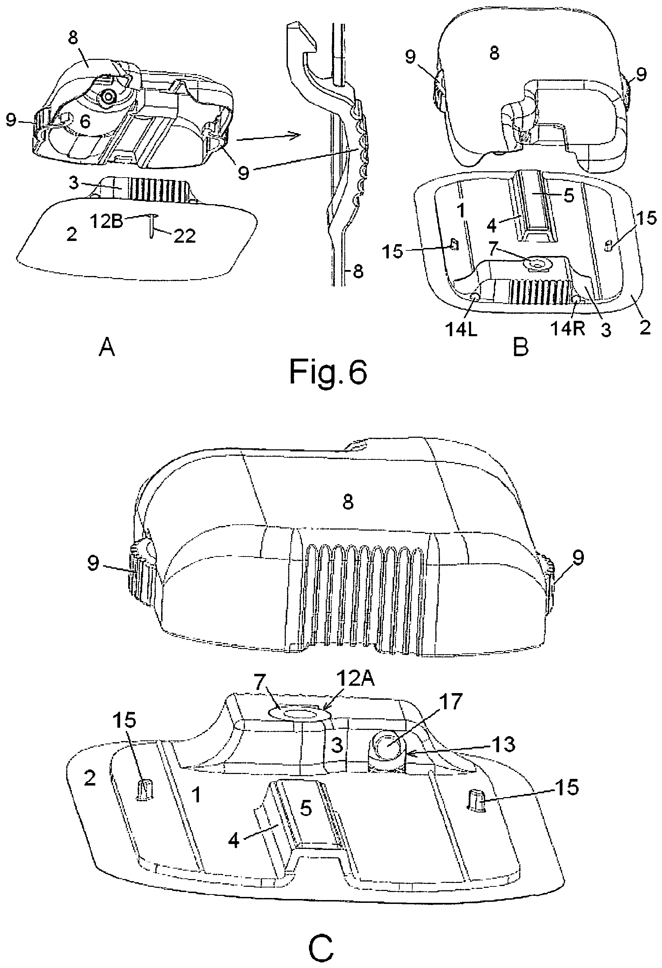

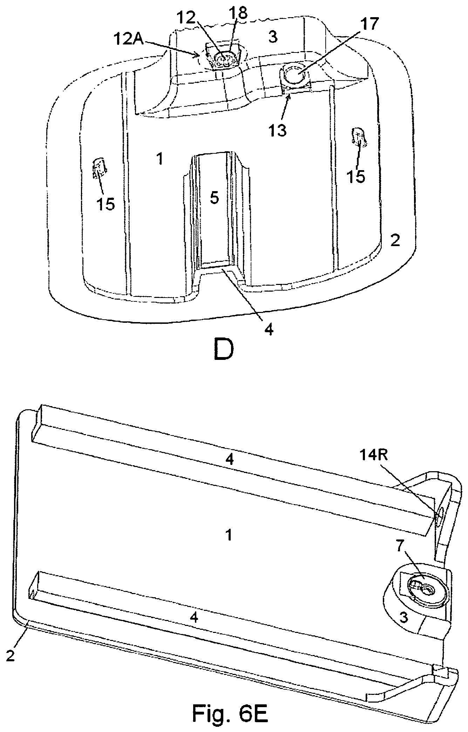

The connector part 3 is kept in position by the surface plate 1. According to one embodiment the surface plate 1 and at least an outer cover of the connector part 3 is simply molded in one piece during manufacturing of the device. The connector part 3 forms a fluid path between e.g. a reservoir 6 of medication or a reservoir for liquid collected from the patient and a cannula part 7. Therefore the connector part 3 is provided with at least two openings, one opening at each end of the fluid path where the first opening 13 is an inlet or outlet opening receiving or delivering fluid to a reservoir 6 and the second opening 12 is an inlet or outlet opening receiving or delivering fluid to a cannula part 7 (see fig. 6C-D). The connection part 3 might be provided with extra openings e.g. for injection of a second medication or nutrient or for letting the fluid in the fluid path get in contact with a sensor. In order to secure a fluid tight connection between the outlet opening 12 in the connection part 3 and the cannula part 7 the outlet opening 12 of the connection part 3 is provided with an elastic sealing 18 around the outlet opening 12. When the cannula part 7 is inserted it will be press fitted into the cannula opening 12 and the elastic sealing 18 will provide a completely fluid tight gasket around the corresponding openings 12 and 20. In order to improved the press-fitting and thereby the fluid tight connection between the cannula part 7 and the outlet of the fluid path, the cannula opening 12A can be provided with a decreasing cross-section in a plane parallel to the cannula 22 when inserted and perpendicular to the surface where the outlet of the fluid path is positioned. The cannula part 7 will have a corresponding decreasing cross-section.

In the following the first opening 13 will be referred to as "inlet" and the second opening 12 will be referred to as "outlet" although the direction of the flow through the fluid path is not significant for the invention.

The connection part 3 is further provided with a cannula opening 12A which accurately fits around a cannula part 7 i.e. the cannula opening 12A has the same shape or profile as the cannula part 7 and is just big enough to let the cannula part 7 pass through and then fit into the opening. When the cannula part 7 is fully inserted into the base part and the patient, then the upper surface i.e. the distal surface of the cannula part 7 is normally at level with or at a lower level than the outer surface of the connection part 3 surrounding the cannula opening 12A. When the cannula part 7 has been fully inserted into the connection part 3, then an opening 20 in a side surface of the body of the cannula part 7 corresponds to the opening 12 of the fluid path of the connection part 3 and fluid can flow from one part to the other.

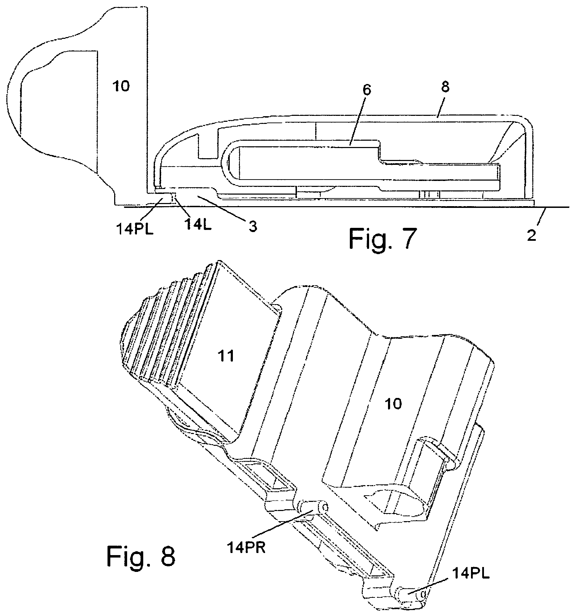

Fig. 5B shows the embodiment of fig. 5A where the inserter has been removed. Fig. 5C shows the same embodiment seen from above but in fig. 5C the penetrating member has been inserted and the inserter has been removed from the assembly. Fig. 5B shows the device from the end which was covered by the inserter 10 before it was removed. From this end it is possible to see a part of the fastening means 14 which assure attachment of the inserter 10 to the base part before insertion. According to this embodiment the fastening means 14 comprise two openings 14L and 14R in the connector part 3. These openings correspond to two protruding parts 14PL and 14PR (see fig. 7 and 8) which protrude from the side of the inserter housing turned towards the base part and the connector part 3 with the corresponding opening. When the fastening means 14L and 14R on the base part is engaged with the corresponding fastening means 14PL and 14PR on the inserter 10, the inserter 10 is prevented from moving in relation to the base part, at least in the direction perpendicular to the surface plate 1. After insertion of the penetrating member where the penetrating member has been fully inserted into the base part, the inserter 10 can be removed or detached from the base part. When detaching the inserter 10 from the base part, the inserter 10 is moved in a direction horizontal to the patients skin i.e. the base part is not subjected to a force perpendicular to the patients skin i.e. a force pulling the base part away from the patient. Alternatively it would be possible to e.g. glue the inserter to the delivery part 8 before insertion along adjoining surfaces between the inserter 10 and the delivery part 8 which surfaces should be essentially perpendicular to the patient's skin in order to create a pull in a direction parallel to the patients skin when the inserter 10 is removed from the delivery part 8.

Fig. 5D shows the reservoir 6 attached to the connection part 3 at the first opening 13 of the connection part 3. I

Figs. 6A-D show the base part and the delivery part in a separated position from different angles. In fig. 6A the two parts are shown from below. This view shows an opening 12B through which the penetrating member 7 can be inserted through the base part and through which opening 12B the cannula 22 extends. From this view it is possible to see how the reservoir 6 can be positioned in the delivery part 8 and to see how two opposite positioned release handles 9 are placed at the edge of the delivery part 8. Further a longitudinal track corresponding to longitudinal raised guiding means 4 on the base part can be seen.

The two release handles 9 are formed as s-shaped bands where one end is fastened hinge-like to the housing of the delivery part 8 and the first curve in the s-shape is slightly extending the outer surface of the housing of the delivery part whereas the second curve is free i.e. not attached to the housing of the delivery part 8 and is provided with a hook-like shape which can fold around a part 15 protruding from the distal surface of the base part. When the delivery part is locked to the base part both release handles 9 are folded round a protruding part 15, when the delivery part 8 is to be removed from the base part, the two opposite release handles 9 are pushed together whereby the hook-like parts of the release handles 9 are released from the protruding parts 15 of the base part, and the delivery part can be moved backwards i.e. in the direction away from the cannula part 7 and removed from the base part in this direction.

In fig. 6B the two parts are shown from above. This view shows how the delivery part 8 of this embodiment can be joined to the base part by pushing the delivery part 8 down toward the guiding means 4 which in this case is a longitudinal raised platform having e.g. a metal lining 5 fastened to the top surface. The delivery part 8 is provided with corresponding means e.g. comprising a track corresponding to the raised platform 4. The corresponding means of the delivery part 8 can slide along the metal lining 5 of the raised platform 4 in the longitudinal direction. When the delivery part 8 arrives at its working position, the two release handles 9 engage respectively with the two protruding parts 15 protruding from the upper surface of the surface plate 1. When the delivery part 8 is in its working position it is locked in all horizontal directions by the release handles 9. The locking mechanisms make it possible to fasten and release the delivery device from the base part as often as needed i.e. a single-use base part can be combined with a multi-use delivery part.

In fig. 6C the two parts are shown from the end opposite of where the inserter was fastened before insertion of the penetrating member. From this side it is possible to see the inlet opening 13 in the connection part 3 through which e.g. medication from the reservoir 6 can enter, the inlet opening 13 is protected with a membrane to prevent contamination with microorganisms. According to one embodiment the connection part 3 is provided with both a connector needle (not shown as it is placed behind the bubble shaped membrane) and a bubble shaped self closing membrane 17 and the reservoir 6 can be provided with a bubble shaped self closing membrane. Hereby a fluid path is established providing transfer of medication e.g. insulin or nutrients from the reservoir to the connector part 3. As both parts are provided with self closing membranes it will be possible to separate the two units from each other and rejoin them at a later time without the connection part 3 and thereby the patient being contaminated.

Fig. 6E shows a second embodiment of the base part. This embodiment is provided with two guiding means 4 in the form of two right angled profiles shaped as: ┐ ┌, and protruding from the surface plate 1 of the base part. The guiding means 4 correspond to means on a delivery part or a cover which is to be fastened to the base part. Such corresponding means can e.g. be formed as one or more hooks having a profile in the form of ┘ and └.

The fluid path of the connection part 3 is very short compared to the embodiment shown in fig. 1-6 and the inlet of the connection part 3 is placed in a centre position in relation to the guiding means 4 but the inserted cannula part 7 has the same profile as the cannula part 7 fitted to the embodiment of fig. 1-6.

Fig. 7 shows a longitudinal cut through an assembly as shown in fig. 4-6. From this view it is possible to the how the fastening means 14 of respectively the connector part 3 of the base part and the inserter 10 are joined together.

Fig. 8 shows the inserter 10 removed from the rest of the assembly. From this side it is possible to see the fastening means 14PR and 14PL of the inserter.

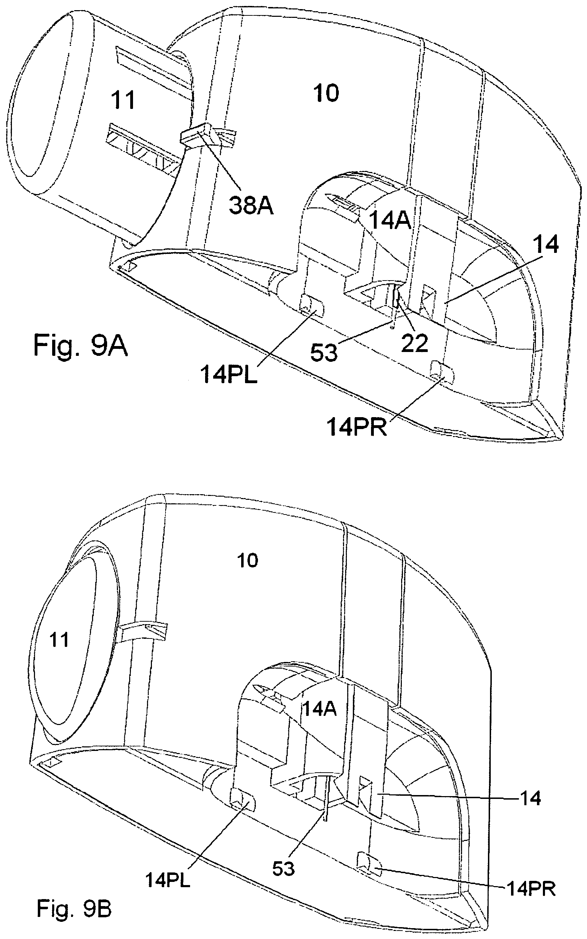

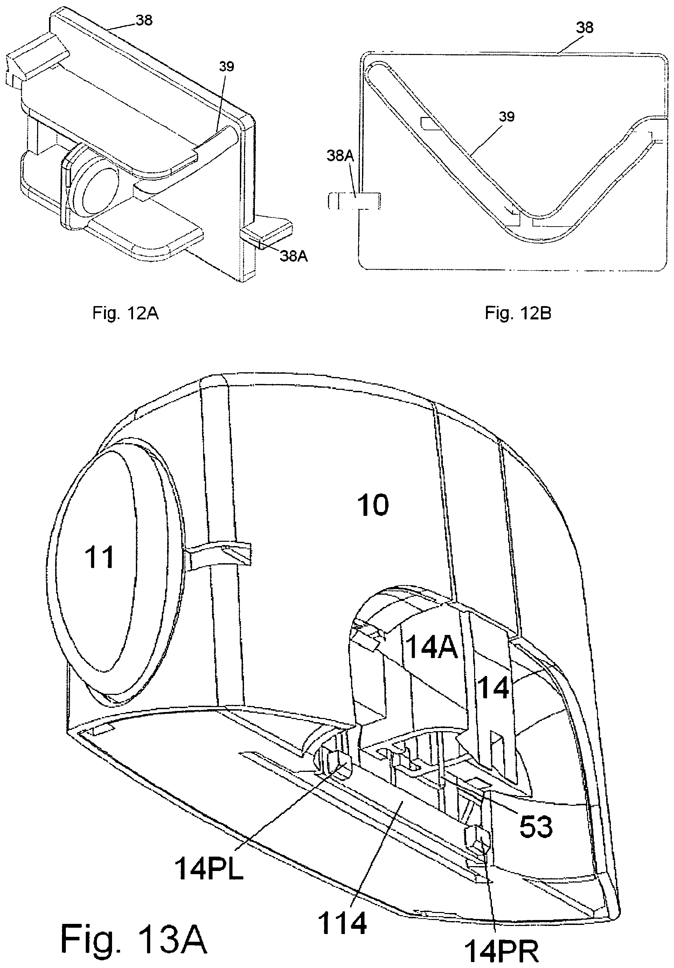

Figs. 9-11 show a third embodiment of an inserter, in fig. 9A and 9B the inserter is shown separated from the rest of the assembly. The inserter 10 comprises like the first and second embodiment of the inserter an actuator handle 11 which in fig. 9A is shown in a pre-insertion state and in fig. 9B is shown in an after-insertion state. The third embodiment of the inserter is provided with a moving part 38 as shown in fig. 12 and this moving part is provided with a protruding member 38A which is an integrated part of the moving part 38. The moving part 38 is shown two different views in fig. 12A and 12B. That it is "an integrated part" means that it moves simultaneously with the moving part and is positioned stationary in relation to the moving part. Normally it will be molded together with the moving part and be of the same material, but it can also be made of a different material and attached to the moving part 38 after the moving part 38 has been produced.

The protruding part 38A on the moving part 38 is provided with a ramp. The ramp is an inclined surface placed on the forward side of the protruding part 38A in such a way that the front profile of the protruding part 38A forms an arrowhead.

The fastening means of this embodiment comprises a hinged part 14 which in this embodiment is fastened to the housing of the inserter 10, the hinged part could alternatively be fastened to an internal part of the inserter e.g. the same part as the protruding parts 14PL and 14PR is fastened to. In the shown embodiment the hinged part 14 is actually made as a part of the housing as the hinged part 14 is created by making two cuts in the full height of the housing. The housing is normally made of a hard, molded plastic such as polypropylene and the relatively long shape of the hinged part 14 makes it very flexible i.e. the hinged part 14 is very pliant and it will be easy to push it outward from the relaxed position, the inward movement is blocked by the presence of the guiding means 33 for the penetrating member which in this embodiment is a cannula part 7. The hinged part 14 can also be made of a material which is different from the material of the housing of the inserter e.g. metal which are then attached to the housing in a rotatable manner.

The hinged part 14 is provided with two inward hooks ("inward" means that the hooks point toward the inside of the housing) at the lower or proximal end of the hinged part 14 and the two hooks lock the housing to the base part by catching a stationary protruding part 14B of the base part. As the two hooks are turned inward they are released from their locked position by being pushed outward i.e. away from the centre of the housing. The hinged part 14 is also provided with a contact member 14A having the form of a rounded plate of a rigid material placed inwards from the hinged part 14 around the guiding means 33 for the cannula part 7. When the moving part 38 moves from its start position to its end position the protruding member 38A which is placed on the trailing edge of the moving part 38 will hit the contact member 14A with the ramp surface and the contact member 14A will be forced outward and so will the hinged part 14 as the contact member 14A is attached unreleasably and rigidly to the hinged part 14.

The housing of the inserter also comprises two protruding parts having the form of rounded hooks 14PL and 14PR on the inside surface of the wall opposite the inward hooks of the hinged part 14. These protruding parts 14PL and 14PR fits into corresponding openings 14L and 14R of the base part close to the connector part 3. The openings in the base part are shown in fig. 16A. When the fastening means in the form of the openings 14L and 14R on the base part is engaged with the corresponding fastening means in the form of the rounded hooks 14PL and 14PR on the inserter 10, the inserter 10 is prevented from moving in relation to the base part, both in the direction parallel to the longitudinal direction of the base part as the protruding parts are rounded and form a grip around the opening, and also in the direction perpendicular to the surface plate 1 due to the insertion of the protruding part into the opening. After having fully inserted the penetrating member (fig. 9B), the inserter 10 can be removed or detached from the base part.

In order to detach the inserter 10 from the base part, the inserter 10 is pivoted around an axis provided along the upper surface of the openings 14L and 14R. The upper (distal) surface of the openings 14L and 14R provide a contact surface for the rounded hooks 14PL and 14PR along which contact surface the downward contact surface of the rounded hooks 14PL and 14PR can slide and thereby be forced out of the openings 14L and 14R when subjecting the inserter housing 30 to a rotational movement. After insertion the base part comprising the surface plate 1 and the inserted part 7 is completely stationary in relation to the surface in which the cannula or sensor has been inserted and the rotational movement is only provided by the inserter 10.

The rotatable movement is made possible at the lower or proximal surface of the housing of the inserter is inclined in relation to the upper surface 1 of the base part and therefore leaves room for the displacement of the housing 30, at the end of the rotational movement the lower (proximal), inclined surface of the inserter housing will normally rest against the patients skin.

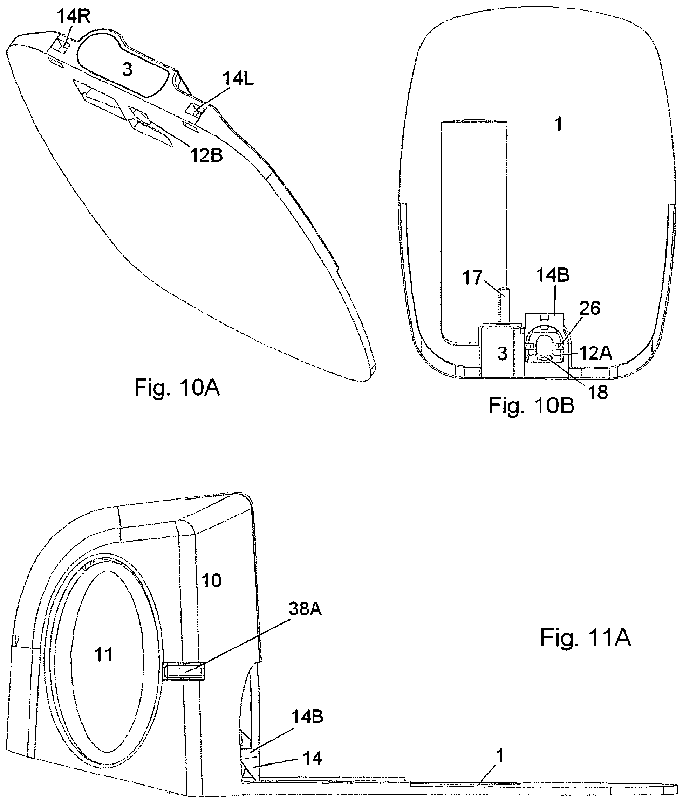

Fig. 10A and 10B show a base part which can be used with the third embodiment of the inserter. Fig. 10A show the openings 14L and 14R, according to this embodiment the openings are rounded in a 90 degree angle and are open toward the proximal surface of the base part i.e. the surface which is placed against the patients skin.

Fig. 10B show the base part seen from above. From this angle it is possible to see down the cannula opening 12A which according to this embodiment is provided with guiding means 26 for the cannula part 7. This guiding means 26 comprise to opposite longitudinal tracks which assure the correct placement of the cannula part 7.

Fig. 11A shows the inserter in a position before insertion. In this state the inclined lower surface is lifted away from the patient's skin. The inward hooks of the hinged part 14 are locked around the protruding part 11B on the base part.

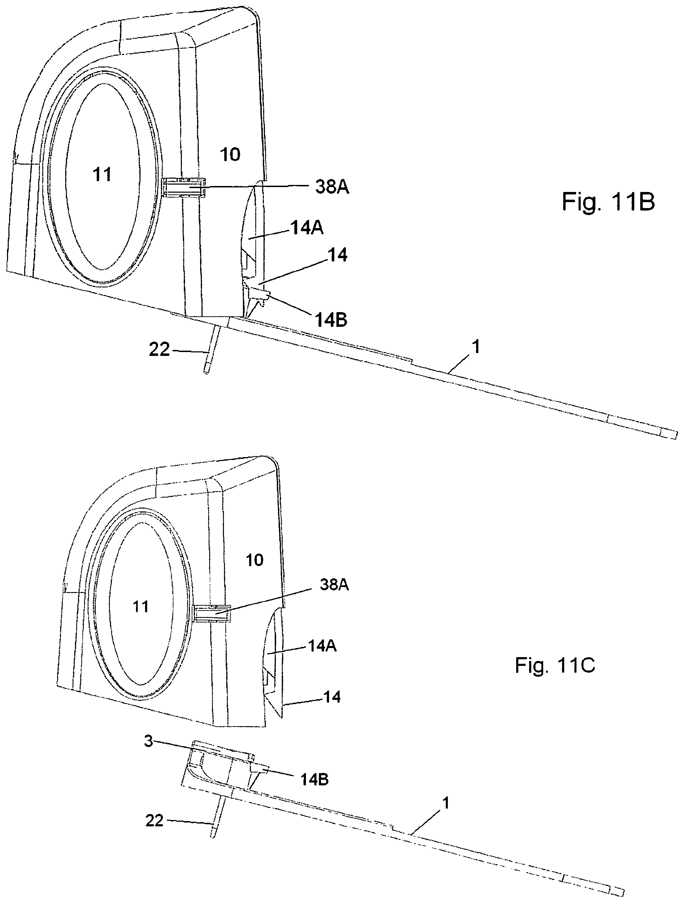

Fig. 11B shows the inserter after the cannula part has been inserted. In this state the inclined lower surface is parallel to the patient's skin and the inward hooks of the hinged part 14 have been released from the locked position.

Fig. 11C shows the inserter after it has been removed from the base part.

Fig. 12A and 12B show the moving part 38 of the third embodiment of the inserter shown in fig. 9-11. Fig. 12A shows the "back side" i.e. the side turned away from the penetrating member and fig. 12B shows the "front side" i.e. the side turned toward the penetrating member. The figures show the protruding part 38A placed at the trailing edge of the moving part 38 having the inclined side i.e. the ramp facing forward in the direction of movement, and the figures show the transformation means 39 in the shape of a longitudinal opening formed as a V where the start position is at the upper end of the first line in the V and the end position for the penetrating member is at the upper end of the second line in the V.

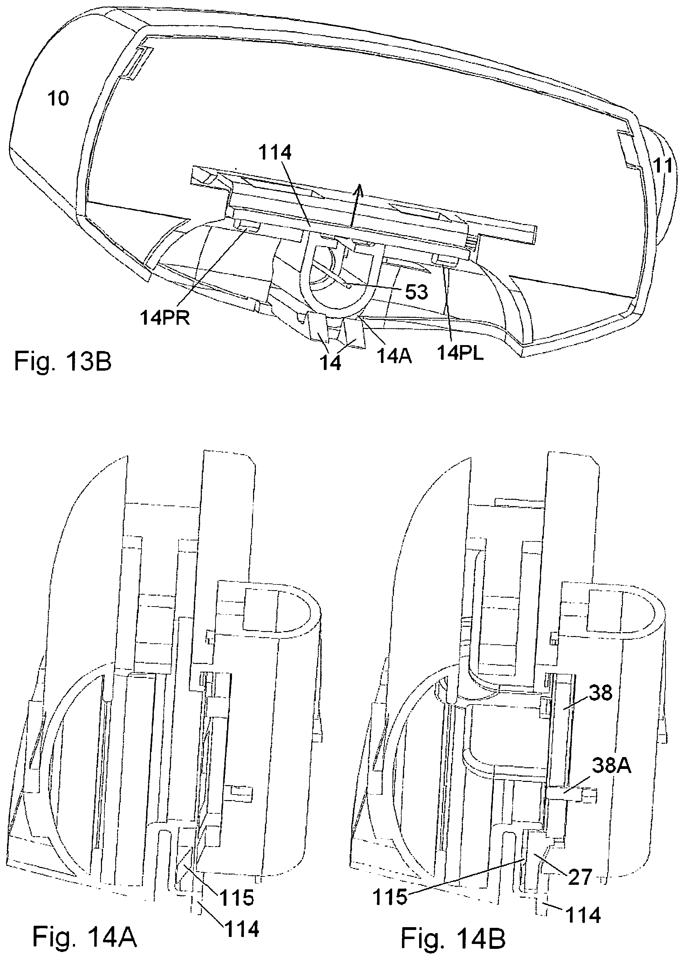

Fig. 13 and 14 show a fourth embodiment of an inserter, this embodiment differs from the third embodiment by the fastening means 14 securing the inserter to the base part. The inserter 10 is in fig. 13 and 14 shown in an after-insertion state where it has been removed from the base part. The fourth embodiment has means to release to sets of functionally independent fastening means which are supporting each other.

Like the third embodiment the fourth embodiment of the inserter is provided with a moving part 38 (see fig. 15A and 15B) having a protruding member 38A being an integrated part of the moving part 38. The moving part 38 of the fourth embodiment is further provided with a second integrated part called the positioning means 27. These positioning means 27 are attached to the lower trailing edge of the moving part 38.

The fastening means of this embodiment comprises like the third embodiment of the inserter a hinged part 14 which is fastened to the housing of the inserter 10 and the hinged part 14 moves in the same way as described for the third embodiment of fig. 9 and 10. The hinged part 14 of the fourth embodiment is also provided with two inward hooks at the lower or proximal end of the hinged part 14. The two hooks lock the housing against the base part by catching a stationary protruding part 14B of the base part having a downward or proximal contact surface. As the two hooks are turned inward they are released by being pushed outward i.e. away from the inside of the housing.

The hinged part 14 is also provided with a contact member 14A having the form of a plate placed in a direction toward the centre of the inserter i.e. "inwards" from the hinged part 14 around the guiding means 33 for the cannula part 7. When the moving part 38 moves from its start position to its end position the protruding member 38A which is placed on the trailing edge of the moving part 38 will hit the contact member 14A with the ramp surface of the protruding member 38A and the contact member 14A will be forced outward and so will the hinged part 14 as the contact member 14A is attached unreleasably and rigidly to the hinged part 14.

According to the fourth embodiment the protruding members 14PL and 14PR are positioned on a flexible member 114. The protruding members 14PL and 14PR according to this embodiment have a rectangular profile but could also have e.g. a round or triangular profile. The protruding members 14PL and 14PR fits into openings 14P and 14L of the base part close to the connector part 3. These openings correspond to the rectangular protruding members 14PL and 14PR. When the fastening means in the form of the openings 14L and 14R on the base part are engaged with the corresponding fastening means in the form of the protruding members 14PL and 14PR on the inserter 10, the inserter 10 is prevented from moving in relation to the base part, both in the direction perpendicular to the surface plate 1 and in any direction parallel to the surface plate 1.

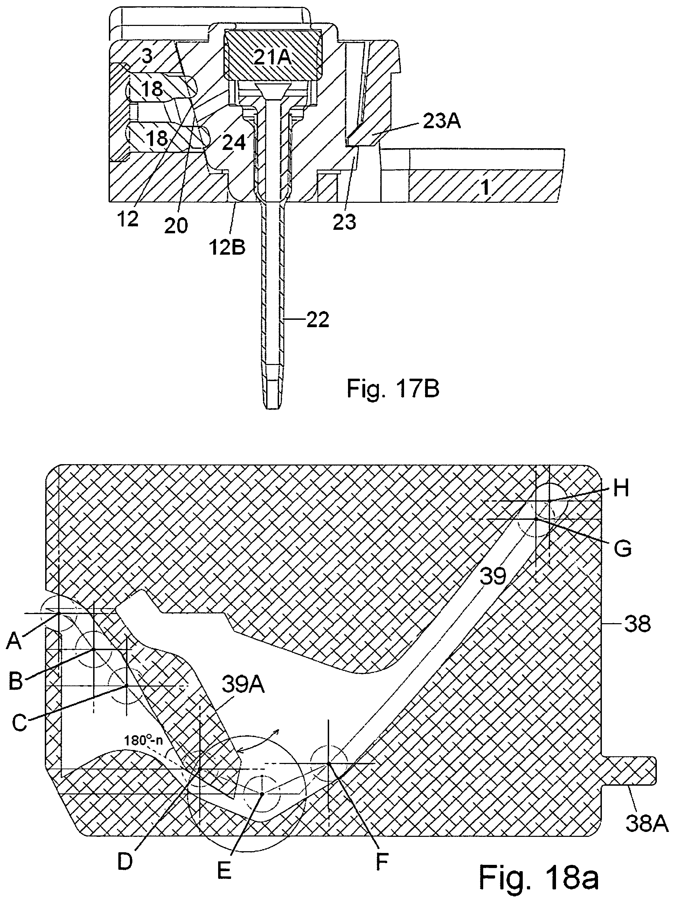

The flexible member 114 is attached to the housing or a part being stationary in relation to the housing 30 in such a way that the flexible member can move between two positions, a first position where the inserter is locked to the base part, and a second position where the inserter is released from the base part. Both fig. 17A and 17B show the flexible member 114 in a relaxed locked position and an arrow in fig. 17B indicates the direction it moves in, in order to get to the second released position. According to the shown embodiment the flexible member 114 is made as an integrated part of the guiding means 32 for the moving part i.e. the flexible member 114 constitutes a part of the surfaces or walls along which the moving part 38 slides. The flexible member 114 is provided with a contact part 115 which according to this embodiment has a triangular profile with the sharpened edge pointing forward in the direction of movement during insertion. The contact part 115 is formed with a ramp shaped surface pointing in the direction opposite of the direction of movement of the moving part 38 during insertion.

In order to bring the flexible member 114 from a first relaxed and locked position into a second and released position the flexible has to be subjected to a force large enough to be able to move the flexible member 114 to the second position.

Fig. 14A and 14B shows the internal parts of the inserter housing 30 which provide the guiding parts for the moving part and which are not visible when the surrounding housing is in place. Fig. 14A and 14B show identical cuts through these internal housing parts but in fig. 14A the moving part 38 is removed in order to make the contact part 115 of the internal parts visible. The contact part 115 consists of a protruding ramped surface which will get in contact with the positioning means 27 of the moving part 38 when the moving part 38 is in its end or final position.

Fig. 15A and 15B show the moving part 38 of the fourth embodiment of the inserter shown in fig. 13-14. Fig. 15A shows the "back side" i.e. the side turned away from the penetrating member and fig. 15B shows the "front side" i.e. the side turned toward the penetrating member. The figures show the protruding part 38A placed at the trailing edge of the moving part 38 having the inclined side i.e. the ramp facing forward in the direction of movement, and the figures show the transformation means 39 in the shape of a longitudinal opening formed as a V where the start position is at the upper end of the first line in the V and the end position for the penetrating member is at the upper end of the second line in the V. The end position is placed lower than the start position. At the lower edge of the moving part 38 is shown positioning means 27 which assures the positioning of the moving part 38 in relation to the housing of the inserter when sliding along the guiding means 32 provided by the surrounding parts of the inserter housing but which main function is to force the flexible member 114 of the housing "backwards" when the moving part 38 and the integrated positioning means 27 passes by.

When the positioning means 27 of the moving part 38 touch the flexible member 114, the flexible member 114 is pushed away from the connection part 3 of the base part, and the fastening means in the form of the protruding parts 14PL and 14PR are pulled out of the corresponding openings in the base part 14L and 14R. When the moving part 38 is in its end position, the integrated parts 38A and 27will be at positions where both the hinge part 14 and the flexible member are pushed away from their relaxed and locked position which means it will be possible to remove the inserter from the base part when the moving part 38 is in its end position.

Fig. 16A shows one embodiment of a penetrating member which can be used with any of the assembly disclosed herein, and fig. 16B and C shows a second embodiment of a penetrating member which can be used with any the assembly disclosed in the present document.

The embodiment of fig. 16A comprises a body 24 provided with a cannula 22 and with a protruding front 25 having a flat surface provided with an opening 20. The protruding front 25 of the cannula part 7 need not be flat; it can actually have any desired shape as long as it is possible to create a corresponding surface on the connection part 3 facing the cannula part 7. In one embodiment the front 25 is inclined in such a way that the cross-section at the upper i.e. distal end of the cannula part 7 is larger than the cross-section at the proximal end of the front, i.e. the end closest to the patient after insertion. The opening 20 of the protruding front 25 is an inlet or outlet through which liquid can enter or exit the cannula part 7. The body 24 is further provided with a top opening 21 which can be covered with a self closing membrane. The opening 21 need some kind of entrance protection as it is facing an outer surface which is in contact with the surroundings. The top opening 21 is primarily used when inserting the cannula part 7 if the cannula 22 is a soft cannula. That the cannula 22 is soft means that it is made of a relatively soft material which can not by it self penetrate the patients skin, in this case it is necessary to use a pointy insertion needle of a relatively hard material when inserting the cannula and this pointy needle can be inserted through the top opening 21, pass through an inner hollow in the body 24 of the cannula part and further pass through the full length of the cannula 22 in such a way that the pointy end of the insertion needle stick out of the open end of the hollow cannula 22. After insertion i.e. after the cannula 22 has been placed sub- or transcutaneous in the patient, then the insertion needle is retracted and the cannula 22 is left inside the patient. The cannula part 7 is also provided with fastening means 23 which in this embodiment has the form of a series of outward hooks 23 which are flexibly fastened to the body 24 in such a way that the hooks can pivot inwards toward the centre of the cannula part 7. When the cannula part 7 is pressed toward the base part, the hooks 23 passes an edge which pushes them toward the centre as they passes the edge and when the hooks have passed the edge they return to their original position and as a upward surface of one or more of the hooks touch a downward surface of the edge the cannula part 7 is locked unreleasably against the edge.

The embodiment of fig. 16B and C comprises the same elements as the embodiment of fig. 16A but this second embodiment is also provided with a guiding track 42 on opposite sites of the body 24 corresponding to protruding parts on the not shown connection part 3. Further the opening 21 to the top placed septum 21A is provided with an upright edge 43 which can help provide an injection site if the user want to perform injections of liquid by a syringe.

Figs. 17A and 17B show an enlargement of a second embodiment of a cannula part 7. Fig. 17A shows the cannula part 7 in a state just before insertion and fig. 17B shows the cannula part 7 inserted into the cavity 12A in the base part. This embodiment also comprises a body 24 provided with a cannula 22 and with a protruding front 25 having a flat surface provided with an opening 20 but according to this embodiment the protruding front 25 is inclined in order to reduce the force required to insert the cannula part and in order to reduce distortion of the sealing 18 while at the same time increasing the pressure between the opening 20 and the sealing 18 around the second opening 12. The inclination of the front 25 is defined by the angle d between the centre line c of the cannula 22 (c is parallel to the insertion direction) and a line parallel to the surface around the opening 20. If the surface around the opening 20 is not straight, then the line parallel to the surface would be the tangent to the surface around the opening 20.The angle d will be larger than 0°and smaller than 90°, normally d ∈ ]0°, 30°] depending on the diameter of the sealing 18 or [60°, 90°[. The distance d₁ between at the distal end of the surface of the protruding part 25, i.e. the end of the cannula part 7 which is furthest away from the patient after insertion, and the centre c of the cannula part 7 is larger than the distance d₂ between the surface of the protruding part 25 at the proximal end, i.e. the end closest to the patient after insertion, and the centre c of the cannula part 7. Normally the distance d₂ will be so small that the proximal end of the protruding front 25 does not touch the sealing 18 of the connection part 3 during insertion.

In one embodiment (not shown) the angle d is close to 90° i.e. d = 90°, such an embodiment would have an upward opening 12, i.e. turned away from the patients skin, in the connection part 3 fitting to a downward opening 20 of the cannula part 7. This means that the force pushing the cannula part 7 toward the sealing 18 will be close to perpendicular to the contact surface of the sealing 18 and this will prevent that the sealing is distorted during insertion of the cannula part 7 by the cannula part 7 sliding along the sealing 18. In another embodiment (e.g. shown in fig. 16) d = 0° as the protruding front 25 and the centre line c are parallel. According to this embodiment the cannula part 7 will be in sliding contact with the protruding sealing 18 which can cause the sealing to be distorted.

The protruding front 25 of the cannula part 7 need not be flat; it can actually have any desired shape as long as it is possible to create a corresponding surface on the connection part 3 facing the cannula part 7. Also the opening 20 of the protruding front 25 can be an inlet or an outlet depending on the purpose of the cannula part 7. In fig. 17A and 17B which is a cut-through view it is shown how the top opening 21 of the body 24 is covered with a self closing membrane 21A. The top opening 21 is primarily used when inserting the cannula part 7, if the cannula 22 is a soft cannula, but the top opening 21 can also be used to inject medication or nutrients other than the primary medication which could be e.g. insulin which the patient receives via the opening 20.

This embodiment of the cannula part 7 is also provided with fastening means 23 and in this embodiment the fastening means 23 has the form of a protruding part 23 on the cannula part 7 which corresponds to a flexible part 23A on the stationary base part. The flexible part 23A can be pushed outward as indicated with an arrow at fig. 17 when the protruding part 23 on the cannula part 7 passes during insertion of the cannula part 7. After insertion the upward surface of the protruding part 23 of the cannula part 7 will be locked by the downward surface of the flexible part 23A of the base part and it will not be possible to detach the cannula part 7 from the base part.

The cannula part 7 of fig. 17A and 17B is provided with a soft cannula 22 which soft cannula 22 together with a bushing 29 provides a cannula assembly. This assembly is normally fastened inside the body 24 of the cannula part 7 by an interference fit i.e. it is only the friction between the body 24 and the cannula assembly which keeps it in the correct position. In order to prevent the cannula assembly from sliding back through the upper larger opening in the body 24 of the cannula part 7, the body 24 of the cannula part 7 can be provided with a ring shaped recess encircling the exit for the soft cannula 22. As the recess creates an open space around the soft cannula 22, the soft cannula 22 can form a small bulk i.e. a ring shaped bulk which prevents the soft cannula from sliding back.

In fig. 17A the height h₂ of the base part 100 is indicated. It is known from the general description of the embodiment that the connector part 3 provides the highest or most protruding part on the base part 100.

Fig. 18a shows another embodiment of the moving part 38 which moving part has an increased tolerance for deviations from the standard insertion depth. Fig. 18a shows the "back side" i.e. the side turned away from the penetrating member and when placed in an inserter the moving part would moved from the right to the left while the penetrating member of the inserter stays in a stationary horizontal position in which position it moves first down and then up. The figure shows the protruding part 38A placed at the trailing edge of the moving part 38, and the guiding means 39 for the transformation means placed within the boundaries of the moving part. According to this embodiment the guiding means 39 are defined by a cutout having an outer limit encircling an open space in which the transformation means 51 of the penetrating member can move. The guiding means 39 also comprise a pivotable part 39A which part can pivot around a stem through which is fastened to the body of the movable part 38. The pivotable part 39A provides a flexible upper limit as the movable part 38 moves from the right to the left according to fig. 18a i.e. the pivotable parts 39A swings upwards as the transformation means passes. When the pivotable part 39A has passed the transformation means 51 of the penetrating member it swings back into its resting position.

The transformation means 51 has a start position relative to the movable part 38 at position A. As the movable part 38 moves to the left, the transformation means 51 of the penetrating member arrive at position B by sliding along the upper surface of the guiding means 39, at position B the insertion needle 53 of the penetrating member touches the skin of the patient.

At position C the cannula 22 which is joined to or surrounding the insertion needle 53 touches the skin of the patient.

At position D the sealing start i.e. contact is made between the cannula part 7 and the surface plate 1, and a retention click can be heard as an information to the user that the cannula 22 is in its correct position and that the retention means 23 on the stationary base part has locked the cannula part 7 to the base part.

As the transformation means 51 of the penetrating member passes from position A to position D it slides along the lower contact surface of the pivotable part 39A. This contact surface drives the penetrating member down and it is therefore important that the surface is smooth and provides as little frictional resistance as possible.

At position E the penetrating member should be fully inserted. That the pivotable part 39A can flex allows for the insertion depth to vary slightly i.e. within the range of ± 0.5 mm.

At position G the insertion needle 53 is clear of the self closing membrane 21A which might cover the top opening 21 of the cannula part 7 and at position H the insertion needle is in a safe position i.e. the insertion needle 53 is retracted relative to the housing of the inserter. Normally it will be retracted at least 1 mm relative to the housing.

As the transformation means 51 of the penetrating member passes from position E to position H it slides along the upward contact surface of the trail which forms the guiding means 39 of the moving part 38. This contact surface drives the penetrating member back up and it should be smooth and provide as little frictional resistance as possible.

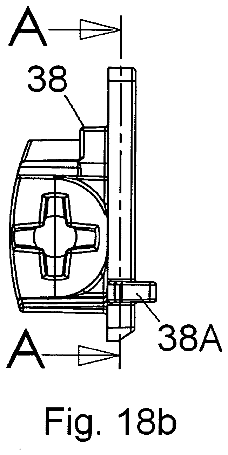

Fig. 18b shows a view of the moving part 38 seen from the side. The arrows marked A indicate the side shown in fig. 18a.

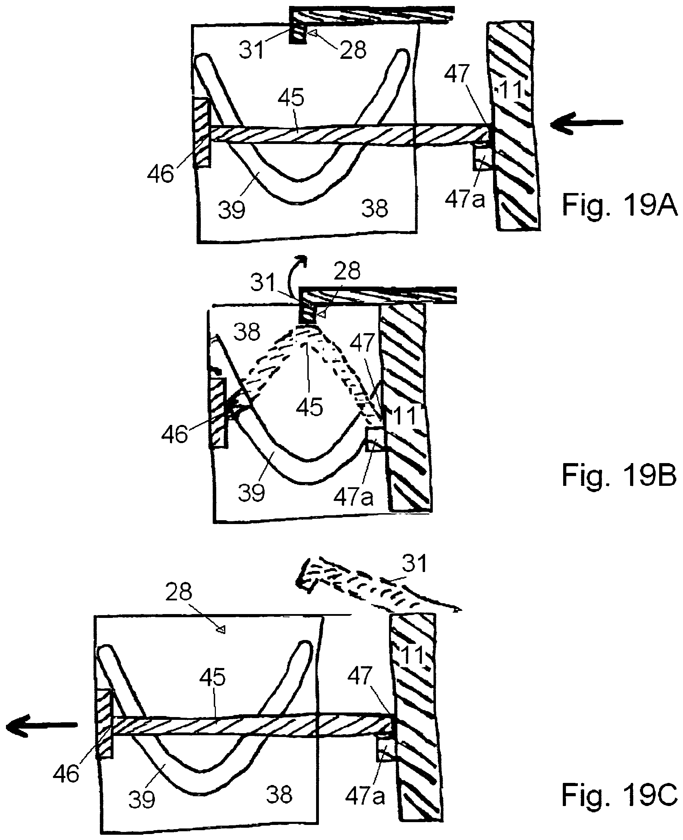

Figs. 19A, B and C show an embodiment of a flat spring which can be used to drive the moving part forward in any of the illustrated embodiments of the inserter. According to this embodiment a spring 45 is provided between the moving part 38 and the activation part 11. Normally the spring 45 will be in a relaxed state during storing as this will normally prolong the time the product can be stored while still being fully functional, if the spring 45 is in a biased state during storing there is a risk that the performance of the product will rapidly decrease. In this embodiment the spring 45 is a flat spring e.g. made of plastic material comprising two ends: a first end 46, attached to, or placed in connection with the moving part 38 and a second end 47 attached to, or placed in connection with the activation part 11. The second end of the spring 45 rests on a block 47a.

The spring 45 of the illustrated embodiment stores energy from the actuation of the of the activation part 11 as the spring 45 is biased through this first movement. A characteristic feature of a flat spring is that when the spring is biased it is bending describing a curve, the presence of the block 47a and the form of the block i.e. the length of the block 47a ensures that the spring 45 can only bend in one direction when it is biased. The not shown housing of the inserter comprises retention means 31. The retention means 31 can have the form of a pivoting arm holding the moving part 38 in a start position by engaging with locking means 28 on the moving part 38. The locking means 28 according to the embodiment illustrated in figs. 19A-C has the form of protruding part with e.g. a triangular or round profile. The deformation of the spring 45 due to biasing can be used to release the moving part 38 from the locked start position.

Fig. 19A shows the embodiment in a start position. The spring is relaxed i.e. unbiased and the retention means 31 of the housing is in a locking position. In order to begin insertion it is necessary for the user o push the actuator 11, by doing this the spring will become biased. During actuation of the activation part 11 the moving part 38 is stationary.

Fig. 19B shows the embodiment in a loaded position. The spring 45 is fully biased and in this fully biased state the spring 45 is curved to such a degree that it touches the retention means 31 of the housing and pushes them away from the locking means 28 of the moving part 38 thereby releasing the moving part 38 from the housing.

Fig. 19C shows the embodiment in a state where the moving part 38 has been moved to its end position. The actuator handle 11 is in the same position as in the fully loaded state of fig. 19B and the retention means 31 of the housing is in an unlocking position. In this state the penetrating member which was to be inserted will be inserted subcutaneously and the next step for the user will be to remove the inserter housing from the insertion site.

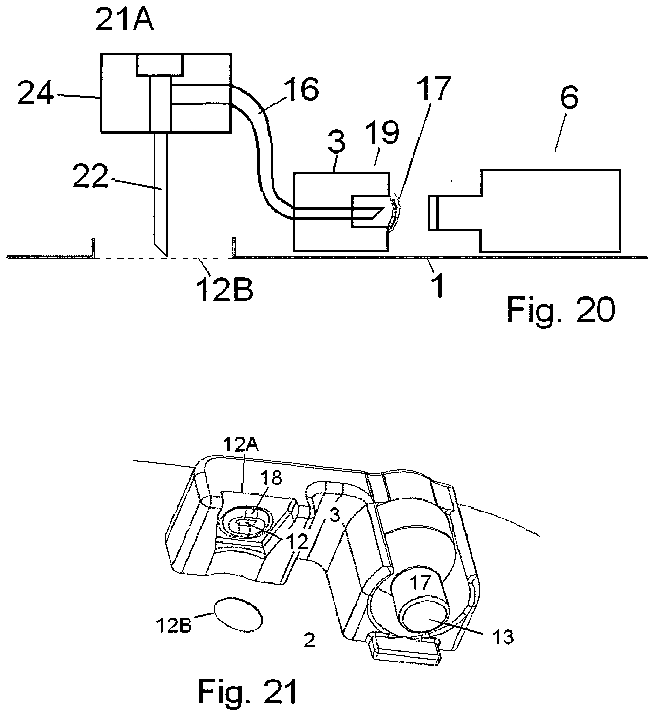

Fig. 20 shows part of an embodiment of an assembly according to the invention. The figure shows the surface plate 1, a penetrating member in the form of a cannula part 7 having a body 24, a cannula 22 and a self closing membrane 21A protecting an upper access of the body 24. The cannula part 7 has a further access opening 20 which is connected to a connection part 3 via a tube shaped connection 16 which connection provides a permanent fluid path between the cannula part 7 and the connection part 3 although the cannula part 7 is not yet placed in a in-use-position. A reservoir containing medication is also shown in a position where it could be situated before it is joined to the connection part 3 via the connector needle 19 and forms a fluid path with the cannula part.

The flexible connection 16 either has to be long and flexible enough to reach between the cannula part and the connection part 3 in both a retracted position and a forward position or the flexible connection 16 has to be elastic and able to be extended in order to let the cannula part get into the retracted position. When the cannula part is in the retracted position it is placed inside an inserter (not shown), it could be an inserter of the type shown in any of the previous figures or it could be a completely different inserter which have the ability to hold the penetrating member to be inserted.

The flexible connection 16 attaches the penetrating member 7 unreleasably to the surface plate 1.

The second embodiment of the base part shown in figs. 6A-6D constitutes an infusion part comprising a cannula part and a fluid path. This embodiment comprises a surface plate 1 attached to a contact surface. The surface plate 1 is in this embodiment constructed of a molded plastic material and the contact surface can be the proximal side of a mounting pad 2 which mounting pad 2 is unreleasably fastened to the surface plate 1 during manufacturing of the device. The mounting pad 2 of this embodiment has the same area as the surface plate 1 but it could be of an area larger or smaller than the surface plate 1.

A connector part 3 is position on the surface plate 1. The connector part 3 provides for the contact between the base part and some kind of delivery means. According to one embodiment the surface plate 1 and at least an outer cover of the connector part 3 is simply molded in one piece during manufacturing of the device. The internal parts of the connector part 3 forms a fluid path between e.g. a reservoir of medication or a reservoir for liquid collected from the patient and a cannula part 7. Therefore the connector part 3 is provided with at least two openings, one opening at each end of the fluid path where the first opening 13 is an inlet or outlet opening receiving or delivering fluid to a not shown reservoir and the second opening is an inlet or outlet opening 12 receiving or delivering fluid to a cannula part 7. The connection part 3 might be provided with extra openings e.g. for inserting the cannula part, for injection of a second medication or nutrient or for letting the fluid in the fluid path get in contact with a sensor. In the following the first opening 13 will be referred to as "inlet" and the second opening will be referred to as "outlet" although the direction of the flow through the fluid path is not significant for the invention.

The fluid path of the connection part 3 of this embodiment is very short and the inlet 13 of the connection part 3 is placed in a centre position in relation to the guiding means 4. The top of an inserted cannula part 7 is shown inserted into the connection part 3.

The connection part 3 is further provided with a cannula cavity 12A which accurately fits around a cannula part 7 i.e. the cannula cavity 12A has the same 3-dimensional shape or profile as the cannula part 7 and is just big enough to let the cannula part 7 pass through and then fit into the opening. In fig. 1 the cannula part 7 is shown in a position where the cannula part 7 is fully inserted. When the cannula part 7 is fully inserted, then the upper surface i.e. the distal surface of the cannula part 7 is normally at level with or at a lower level than the outer surface of the connection part 3 around the cannula cavity 12A.

When the cannula part 7 has been fully inserted into the connection part 3, an opening 20 in a side surface of a body 24 of the cannula part 7 corresponds to the opening 12 of the fluid path of the connection part 3 and fluid can flow from one part to the other. The opening 12 might in the following be referred to as an "outlet" although the direction of the flow is not significant to the invention.

A delivery part corresponding to this embodiment could be joined to the base part by pushing the delivery part down toward the guiding means 4 which in this case is a longitudinal raised platform having a magnet 5 fastened to the top surface. The delivery part would be provided with a corresponding magnet e.g. of a smaller or different size than the magnet 5 which is placed in such a way e.g. in a track corresponding to the raised platform 4, that the corresponding magnet of the delivery part can slide along the magnet 5 on the raised platform 4 of the base part in the longitudinal direction. When the delivery part arrives at its working position, two release handles can engage respectively with two protruding parts 15 protruding from the upper surface of the surface plate 1. When the delivery part is in its working position it is locked in any horizontal direction by the release handles and in the direction perpendicular to the surface plate 1 by the two corresponding magnets of respectively the delivery part and the base part. These locking mechanisms make it possible to fasten and release the delivery device from the base part as often as needed i.e. a single-use base part can be combined with a multi-use delivery part.

Normally the inserter 10 holds the cannula part 7 before insertion and the insertion can be initiated by pushing a handle 11. After insertion a not shown insertion needle can be retracted to the inside of the inserter 10 and the inserter 10 might be removed from the base part, leaving an inserted cannula 22 fastened to the surface plate 1. If the cannula 22 of the cannula part 7 is a hard self penetrating cannula there will be no separate insertion needle and therefore no need to retract the insertion needle.

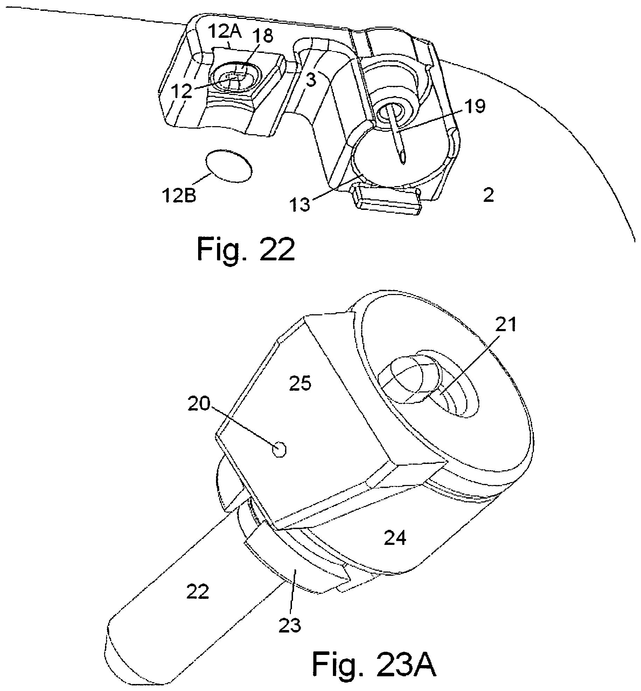

In figs. 6A-6D the connection part 3 is shown with an outer cover provided by the molded surface plate 1. The outer cover shown in this embodiment is not an independent unit but is attached unreleasably to or simply made as a part of the surface plate 1 e.g. by a molding process. The outer cover is provided with a cannula cavity 12A for the cannula part 7 and an access opening 13 for e.g. a reservoir thereby allowing access to the fluid path of the connection part 3 by the reservoir and the cannula part 7. The cannula cavity 12A allows the cannula part 7 to be inserted sub- or transcutaneously into the patient within the circumference of the hard surface plate 1, and the contact surface 2 of the base part, which according to this embodiment is provided by a mounting pad 2, is also provided with an opening 12B which allows for the cannula to be inserted (see fig. 21 and 22). This opening 12B is not necessary if the contact surface 2 is constructed of such a material and thickness that it can be penetrated by at least the cannula 22 of the cannula part 7.

In figs. 21 and 22 the connection part 3 is shown without the outer cover provided by the molded surface plate 1. In order to secure a fluid tight connection between the outlet opening 12 in the connection part 3 and the cannula part 7 the outlet opening 12 of the connection part 3 is provided with an elastic sealing 18 around the outlet opening 12. When the cannula part 7 is inserted it will be press fitted into the cannula opening 12 and the elastic sealing 18 will provide a completely fluid tight gasket around the corresponding openings 12 and 20. In order to improved the press-fitting and thereby the fluid tight connection between the cannula part 7 and the outlet of the fluid path, the cannula cavity 12A can be provided with a decreasing cross-section in a plane parallel to the cannula 22 when inserted and perpendicular to the surface where the outlet of the fluid path is positioned. The cannula part 7 will have a corresponding decreasing cross-section.

In order to secure a fluid tight connection between the inlet opening 13 in the connection part 3 and the reservoir 6, a bubble shaped membrane 17 has been positioned around the first opening 13. The membrane 17 completely covers the inlet opening 13 and prevents contamination of the internal of the connection part 3. When a reservoir or connecting parts for a reservoir is pressed towards the connection part 3, a connector needle 19 will penetrate the membrane 17 and provide a completely fluid tight transfer of fluid between the connection part 3 and the reservoir.

That the membrane 17 is bubble shaped means that it is attached around the opening - normally around the edge of the opening - it protects and the membrane 17 protrudes from the planed formed by the edge of the opening and forms a dome in a distance from the edge which distance normally corresponds to the length of a connector needle 19.

In fig. 21 the connector needle 19 is shown as being a part of the connection part 3 i.e. it is attached to the connection part 3 but it might just as well be a part of the reservoir.

The connection part 3 might be provided with both a connector needle 19 and a bubble shaped self closing membrane 17 and the reservoir might also be provided with a bubble shaped self closing membrane. As both parts are provided with self closing membranes it will be possible to separate the two units from each other and rejoin them at a later time without the internal fluid path of the connection part 3 and thereby the patient being contaminated.

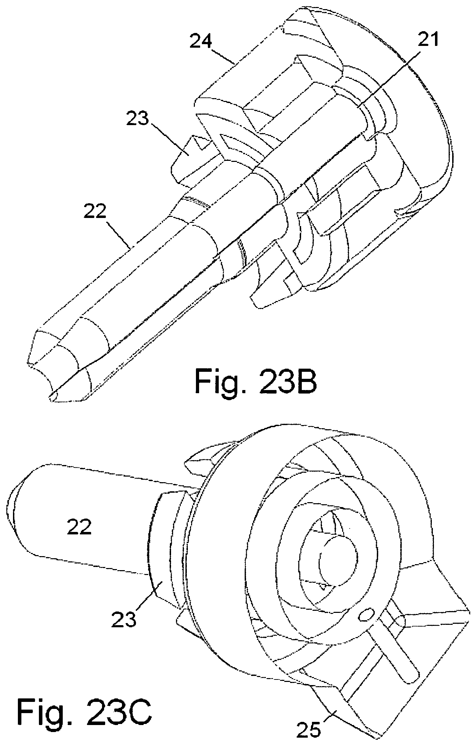

Fig. 23A, 23B and 23C shows an enlargement of a cannula part 7 which can be used in connection with the invention. This embodiment comprises a body 24 provided with a cannula 22 and with a protruding front 25 having a flat surface. The surface of the cannula part 7 having an opening need not be flat; it can actually have any desired shape as long as it is possible to create a corresponding surface on the connection part 3 facing the cannula part 7. In one embodiment the front 25 is inclined in such a way that the cross-section at the upper i.e. distal end is larger than the cross-section at the proximal end, i.e. the enc closest to the patient after insertion, of the front in at least one dimension. The front 25 is provided with an opening 20 through which liquid can exit or enter the cannula part 7. The body 24 is further provided with a top opening 21 which opening can be covered with a self closing membrane. The opening 21 need some kind of entrance protection as it is facing an outer surface which is in contact with the surroundings. The top opening 21 is primarily used when inserting the cannula part 7 if the cannula 22 is a soft cannula. That the cannula 22 is soft means that is made of a relatively soft material which can not penetrate the patients skin, in this case it is necessary to use a pointy insertion needle of a relatively hard material when inserting the cannula and this pointy needle can be inserted through the top opening 21, pass through an inner through going opening in the body 24 of the cannula part and further pass through the full length of the cannula 22 in such a way that the pointy end of the insertion needle stick out of the open end of the hollow cannula 22. After insertion i.e. after the cannula 22 has been placed sub- or transcutaneous in the patient, then the insertion needle is retracted and the cannula 22 is left inside the patient.

The cannula part 7 is also provided with fastening means 23 which fastening means 23 lock the cannula part 7 to the base part at the time where it is fully inserted. The fastening means 23 of this embodiment comprises outward hooks that can pivot around an axe close to the body 24 of the cannula part 7 in such a way that the diameter formed by the outermost edge of the hooks can be reduced when the hooks are pressed inward i.e. towards the centre of the cannula part 7. When the pressure is removed the hooks will return to their original position due to the flexibility of the material. The hooks will be pushed inwards when they pass an opening such as e.g. the opening 12B or a corresponding opening in the surface plate having a cross-section which at least in one dimension is smaller than the outer edge of the hooks and as the hooks return to their original position after having passed through the opening, the hooks will lock the cannula part 7 in the inserted position.

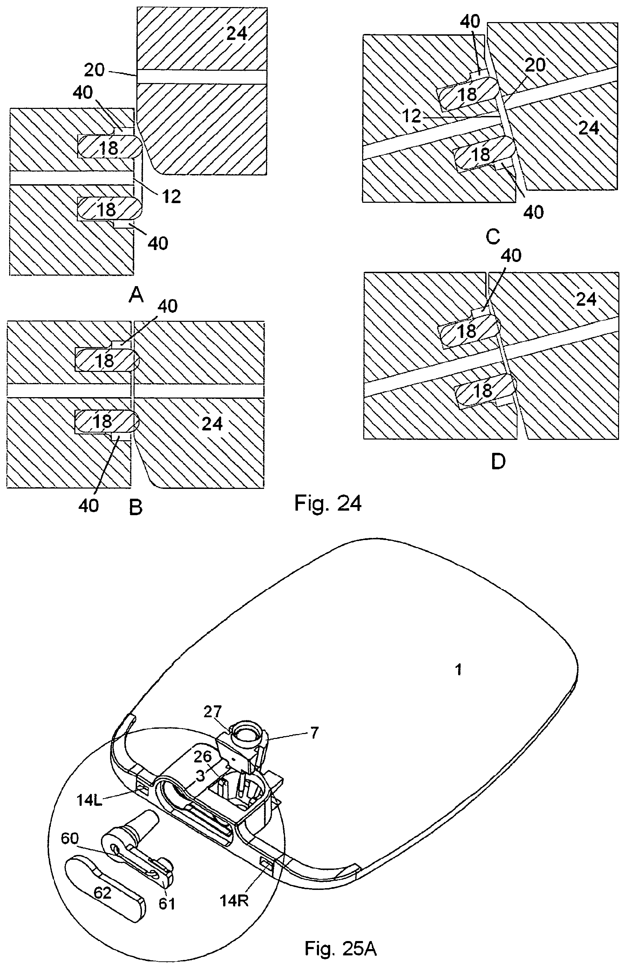

Fig. 24 illustrates how the unrestricted openings between the cannula part 7 having the body 24 and the fluid path having the inlet/outlet opening12 slide into place. Fig. 24A and 24B show an embodiment where d = 0°and fig. 24C and 10D show and embodiment where d is around 15°, normally between 8-22°. According to the embodiment of fig. 24A and 24B the body 24 of the cannula part 7 is provided with an inclined edge in order to reduce distortion or tearing of the sealing. In both embodiments the shown sealing 18 is a circular or cylindrical silicone unit which is placed in a round track around the inlet/outlet opening 12 in the connection part 3. The wall where the sealing or gasket 18 has been placed is provided with an adjacent expansion room 40. After positioning of the cannula part 7 the sealing 18 can occupy this room. In the embodiment of fig. 10C and 10D is not only the sealing face angled, the whole cylindrical sealing part 18 is angled in order to allow uniform sealing deformation. The cylindrical sealing 18 does not form the walls of the inlet/outlet opening 12, the wall or surfaces of this opening is formed by the material which the connection part 3 is formed of in order to provide a pipe which cannot be deformed. In order to create the necessary pressure between the seal and the seal face i.e. the surface which the sealing 18 touches when in a sealing position, the sealing face can be provided with a small continuous protrusion protruding from the sealing face and having the same shape as the sealing which would e.g. be circular if the sealing has the cylindrical shape shown in fig. 24A-D.

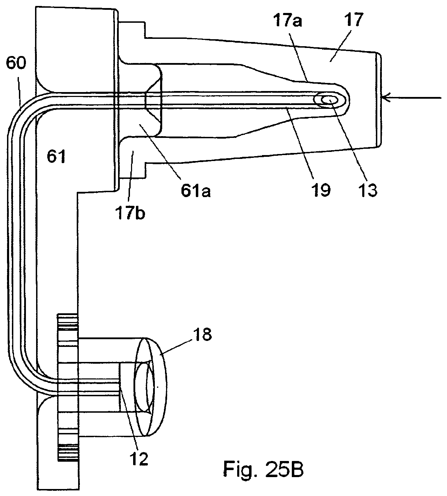

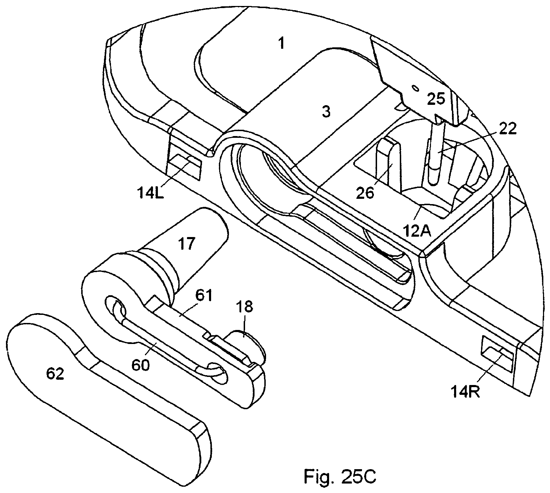

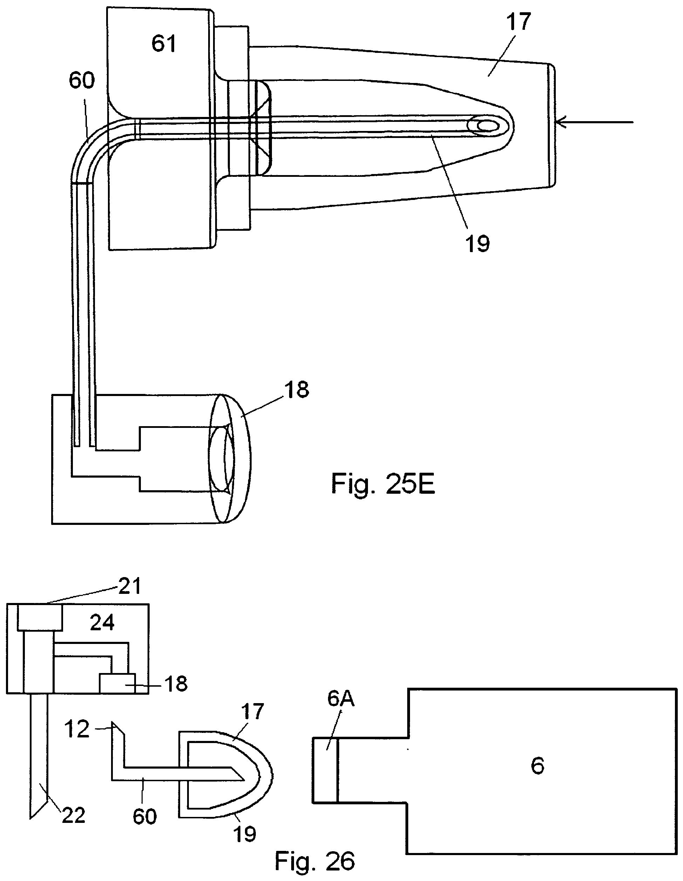

Figs. 25A-25C show one embodiment of a connection part 3. Fig. 25A show the embodiment of the connection part 3 in an exploded view where the internal holding parts 61 for a tube 60 providing a fluid path is shown. Fig. 25B shows a cut through the internal holding part 61 according to which it is possible to the position of the tube 60. Fig. 25C shows an enlargement of the encircled part of fig. 25A.

According to the present embodiment the connection part 3 and the surface plate 1 is molded in one piece of a plastic material, the connection part is provided with several openings, one opening is the cavity 12A which is prepared for fitting in the cannula part 7 and another opening is prepared for fitting in the internal parts of the connection part 3. The internal parts of the connection part 3 according to this embodiment comprises one tube which at two positions are bend in 90° i.e. both the inlet and the outlet end of the tube 60 points in the same direction perpendicular to the connecting part of the tube 60 where the connecting part of the tube 60 forms the fluid path between the two bending parts.

At one end the tube 60 is protected by a bubble shaped membrane 17 and at the other end the tube 60 is open and unprotected, but the open tube end is surrounded by a sealing 18 which is attached unreleasably to a holding part 61. When the internal parts have been placed in the corresponding opening in the connection part 3 a cover 62 accurately fitting in the opening is placed in level with the surface of the connection part 3 in such a way that the user experience a smooth surface which cannot be tampered with.

The embodiment of the base part shown in fig. 11A is provided with guiding means 26 placed inside the cavity 12A of the connection part 3. The two opposing ribs 26 which constitute the guiding means correspond to closely fitting openings 27 in the cannula part 7. The guiding means 26 and the corresponding parts 27 on the cannula part can have other forms, the important feature is that they correspond to each other and make it possible for the cannula part 7 to slide into use position.

Fig. 25B shows an enlargement of the internal parts of the connection part 3. The holding parts 61 comprise a single molded part which is providing a stable embedment of the tube 60. The open end of the tube 60 opens into a volume surrounded by the sealing 18. The closed end of the tube 60 opens into a volume which volume is completely surrounded by an elastic membrane 17. "Completely surrounded" means that there is no free access to the surroundings, "elastic membrane" means that the membrane can be deformed and return to the original form and that the membrane can be penetrated by a needle, especially the connector needle 19 which is provided by the end of the tube 60 and is adapted to penetrate the membrane 17. The membrane 17 is fastened to the holding part 61 as the elastic material at the open end 17b of the membrane 17 squeezes around the protruding part 61a of the holding part 61. The end of the tube 60 which constitutes the connector needle 19 is in this embodiment not in touch with the surrounding membrane 17 when the membrane is not subjected to a pressure from the outside. The connector needle 19 is surrounded by air, and the internal space surrounding the connector needle 19 has a several zones of cylindrical or conical shape i.e. a circular cross-section. The first zone closest to the holding part has approximately both inner and outer cylindrical having walls of approximately constant thickness. The second or middle zone has inner and outer walls formed as truncated cones and the variations of the inner and outer walls result in that the walls have a decreasing thickness towards the holding part 61. The third zone which can also be referred to as a passage 17a is closest to and surrounding the tip of the connector needle 19, both the inner walls and the outer walls of this zone are slightly cone-shaped but could as well be cylindrical or have an angular cross-section, the walls in this zone is thick although having slightly decreasing thickness towards the middle zone, the end of the zone is closed with a flat layer of membrane. When the a pressure is put on the flat end layer, the walls of the first zone of the membrane 17 will deform by bending inwards or outwards when the length of the membrane 17 is reduced as a result of the applied pressure. As the membrane 17 is placed behind the opening 13 when liquid is transferred e.g. from a reservoir to the connector needle 19, fluid will not be present in the volume surrounding the connector needle 19. When the pressure is removed and the membrane 17 returns to the position in front of the opening 13, fluid will normally not run out of the connector needle 19 as the connector needle 19 has a relatively small diameter (< 1 mm) and there is no free access of air at the cannula end of the tube 60.

Fig. 25C shows an enlargement of the enclosed field marked in fig. 25A. The holding parts 61 comprise a single molded part which provides a stable embedment of the tube 60 but in this embodiment the holding part 61 is circular or cylindrical and a non-rigid sealing part 18 is attached to the blunt end of the tube 60 i.e. the open blunt end of the tube 60 opens into a space surrounded by sealing material. The closed end of the tube 60 which is - as in the embodiment of fig. 25B - pointy is completely surrounded by a soft membrane 17 and the holding parts 61 provide the internal parts with enough stability to push the assembled internal parts into position in an adapted opening in the connection part 3. For all embodiments "Completely surrounded" means that there is no free access to the surroundings, and "soft membrane" means that the membrane can be penetrated by a needle, especially the connector needle 19 which is provided by the end of the tube 60 and which is embedded inside the soft membrane 17.

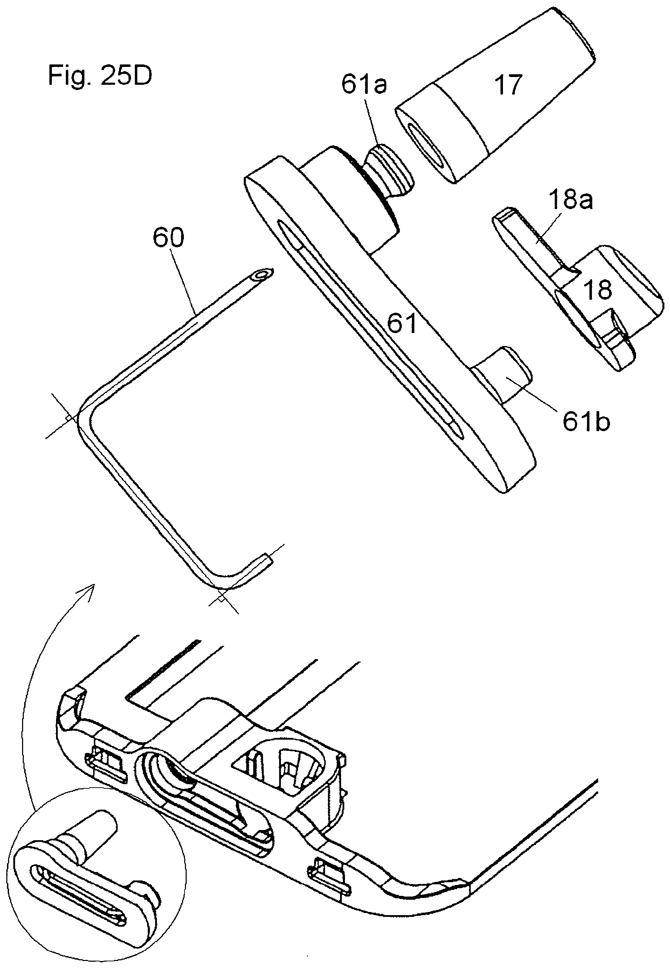

Fig. 25D shows another embodiment of the connection part compared to the connection part shown in fig. 25A-C, although the connection part 3 comprises the same units. Fig. 25DA shows an exploded view of the internal parts of the connection part 3 which internal parts are encircled below in the figure.

Like in fig. 25A-C the holding parts 61 comprise a single molded part. The holding part 61 provides a stable embedment of the tube 60, the holding part 61 is normally molded in one part but it might be formed by joining two or more smaller parts. Such smaller parts could be joined by welding or gluing. As the holding part 61 is rather small, normally less than 2 cm in length, it can be difficult to join the smaller parts. The tube 60 has two open ends, i.e. liquid can pass in or out, and when the tube 60 is mounted in the holding parts 61, the first open end opens into a space surrounded by the closed soft membrane 17 and the second open end opens into a space surrounded by the sealing 18.

The first end of the tube 60 is pointed i.e. sharp and can provide a connection to the reservoir 6 as this first end of the tube 60 can penetrate both the closed soft membrane 17 surrounding the open end of the tube 60 and a membrane 6A protecting the inlet to the reservoir 6. Like in the embodiment shown in fig. 25A-C this end of the tube 60 is completely surrounded by a soft membrane 17 where "completely surrounded" means that there is no free access from the first open end of the tube 60 to the surroundings, "soft membrane" means that the membrane can be penetrated by a needle, especially the connector needle 19 provided by the end of the tube 60. The end of the tube 60 which constitutes the connector needle 19 is in this embodiment not in touch with the surrounding membrane 17 when the soft membrane 17 is not influenced by impacts from the surroundings. The soft membrane 17 is according to this embodiment fastened to the holding part 61 by pressing the relatively soft and compliant membrane material against the holding part 61, the edge of the membrane 17 being closest to the holding part 61 can expand in diameter and slide over a mushroom shaped fastening part 61a which is an unreleasable part of the holding parts 61. When the soft membrane 17 is in its final position, the extended diameter of the membrane 17 can return to a smaller size and this reduction of the diameter will keep the membrane 17 in place around the fastening part 61a. The fastening of the membrane 17 is enhanced if the membrane 17 is provided with one or more inward protruding parts which will rest against the part of the fastening means 61a being closest to the holding parts 61 and having the smallest diameter after mounting of the membrane 17.

The second open end of the tube 60 is blunt and opens into a closed ring of sealing 18 i.e. the sealing has the form of a short pipe and do not stop the flow of liquid in or out of the tube 60. The sealing 18 is fastened to the holding parts 61 by fastening means 18a, the fastening means 18a makes it easier to e.g. weld or glue the sealing 18 unreleasably to the holding part 61.

The tube 60 is formed in one piece; normally it will be made of steel or a hard plastic material. If the tube is formed with a pointed end which is to penetrate the soft membrane 17 during use, it should at least be made of a material which is hard enough to penetrate the soft membrane 17 and e.g. the membrane 6A covering the inlet to the reservoir 6. It is possible to construct the tube 60 with two blunt ends, according to such an embodiment the reservoir 6 could be provided with a connector needle 19 which could penetrate the soft membrane 17 when transferring liquid to the cannula part 7.

According to the embodiment of fig. 25D, the tube 60 is bended at two positions. This is suitable according to this embodiment as the reservoir 6 and the cannula part 7 are mounted on the same side relative to the holding parts 61. The angles of both the bends are 90 degrees, if the tube 60 is to be positioned in a one piece holding parts 61 by pushing, then the two legs provided by these two bends should have the same angle in relation to the connecting tube piece between the two bends but the angles need not be 90 degrees. If the reservoir 6 and the cannula part 7 are positioned different in relation to each other the tube 60 might be bend only once e.g. in the situation where the cannula part 7 is positioned close to the edge of the surface plate 1 and has the front provided with the opening 20 turned toward the first and only bending of the tube 60.

According to one embodiment the tube 60 comprises a hollow needle e.g. made of steel. Such a needle can easily be manufactured at an automated process at a low price. Also such a needle can easily be bending in one or more positions in order to satisfy any need there would be for positioning of the needle between the reservoir 6 and the cannula part 7. Whether the needle is provided with blunt or pointed ends can depend on the parts corresponding at the ends of the needle but normally the needle will be provided with at least one pointed or sharp end which is able to penetrate a protective membrane.

Also if the connection part 3 is placed on a middle or central part of the surface plate 1, then the reservoir 6 could be placed at one side of the connection part 3 at the first end of the tube 60 and the cannula part 7 could be placed at the opposite side of the connection part 3 at the second end of the tube 60 and then the tube 60 could be straight without any bending.

According to the present invention the tube 60 is stationary relative to the surface plate 1 after the tube 60 has been positioned in the holding part 61 and mounted on the surface plate 1. That the tube 60 is stationary means that it does not pivot or in any way move back or forth in relation to the surface plate 1, the tube 60 simply serves as a path for transporting liquid.

Fig. 26 shows an embodiment of an infusion part where the angle d = 90°. The inlet/outlet opening 12 is constructed as a pointy end of a tube 60 which provide for the fluid path or connection between the reservoir 6 and the cannula part 7. A membrane e.g. self closing protects the entrance to the reservoir 6 which means that micro organisms cannot access the reservoir 6 when the reservoir is removed from the connection part 3.

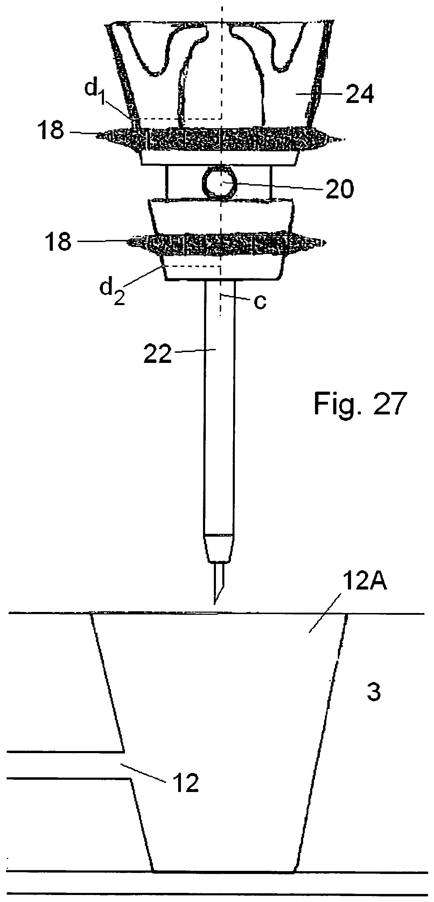

Fig. 27 shows yet an embodiment of a cannula part 7 which can be used with an infusion part according to claim 1. The body 24 of the cannula part 7 has the shape or profile of a truncated cone i.e. in each horizontal (according to fig. 13) cross-section of the body it is round having varying diameters. The body 24 is provided with two permanently attached circular sealings or gaskets 18. Between these two gaskets 18 is the opening 20 positioned which opening 20 allows for fluid to enter the inner through going opening of the cannula part 7. The cannula part 7 is to be placed in a below illustrated connection part 3 provided with a corresponding cavity 12A also having the shape of a truncated cone. The cavity 12A has an inlet/outlet opening 12 for fluid flowing to or from the cannula 22.

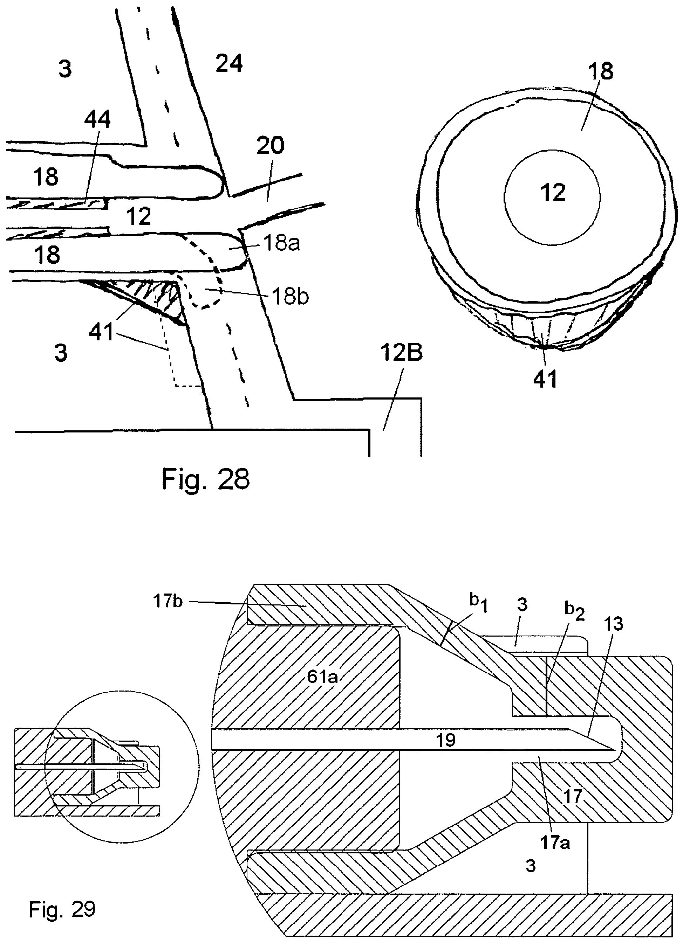

Fig. 28 shows an embodiment of a sealing 18, both as a cut-through view from the side and as a front view from the entrance of the opening 12, between the opening 20 in a side surface of the body of e.g. a cannula part 7 and the opening 12 of the fluid path of the connection part 3. The sealing 18 according to this embodiment has the form of an O-ring i.e. a cylindrical tube attached to or pushed into the connector part 3 encircling the opening 12. The sealing 18 is provided with an inner support 44 which according to this embodiment has the form of a cylindrical tube.

When the cannula part 7 is inserted into the opening 12A the sealing 18 might be distorted due to the tight fit of the cannula part 7, due to the tight fit the inserted part, here the cannula part 7, will touch and slide along the sealing. This movement can cause the sealing 18 to get pulled out of position and when the sealing 18 is pulled out of position it might either cause liquid to leak or the inserted part to jump back thereby pulling the subcutaneously positioned part away from the desired position. In fig. 28 the sealing 18 is shown in two positions: the first position 18a is the desired position where the sealing 18 stops leaking, in the second position 18b the sealing is caught between the down-moving body 24 of the inserted part and the surface of the connector part 3.

One solution to this problem is to lubricate the sealing e.g. with silicone or otherwise ensure that the sealing 18 is very smooth, a second solution would be to lubricate the part to be inserted, here the cannula part 7, and a third solution would be to provide a bevelled edge 41 below the lower edge of the sealing 18. Such an opening can be provided by cutting of the edge below the sealing as illustrated in fig. 28 or as illustrated by dotted lines by cutting of a corner and thereby increasing the distance between the inserted part 7 and the connector part 3 below the sealing 18 by "moving" the surface of the connector part 3 to the left.

Fig. 29 shows an enlargement of an embodiment of a membrane 17 to be used with the invention. This bubble membrane 17 completely surrounds the part of the connector needle 19 which protrudes from the surface of the holding part 61 in which the connector needle 19 is embedded. According to this embodiment the connector needle 19 does not touch the bubble membrane 17 when no pressure is put on the membrane 17 i.e. the connector needle 19 is completely surrounded by air which makes it possible to gas sterilize the connector needle 19; this is the state in which the membrane is shown in the figure. At the proximal end of the membrane 17 in a first area, the membrane 17 provides a passage 17a inside the membrane 17 having a reduced cross-section compared to a middle area of the membrane 17 surrounding a middle section of the needle 19. The passage 17a is defined by the inner surfaces of the walls of the membrane 17. The tip of the connector needle 19 is surrounded of quite thick walls constituted of the membrane forming a small air filled room around the tip of the connector needle 19 and pro-viding the membrane material with a certain rigidity, while a middle part of the membrane closer to but free of the holding part has walls of approximately half this thickness providing flexibility of the walls, this has the result that when pressure is put on the membrane the thick walled part does not change shape, in stead the part of the membrane having reduced wall thickness i.e. the part closest to the holding part will give in, fold inward or outward, while the thick walled part is pressed toward the holding part 61. The membrane 17 further is provided with an open end 17b closest to the holding part 61; this open end 17b of the elastic membrane material fits around a protruding part 61a of the holding part 61. Due to friction between the inner surfaces of the open 17b end of the membrane 17 and the protruding part 61a, the membrane 17 stay put in the desired position i.e. the membrane is kept in position as a result of the interference fit.

Generally a membrane 17 according to the present invention surrounds a needle 19 and comprises three separate and identifiable areas which areas can be defined relative to the needle 19:
- a first area surrounds the tip of the needle 19, this area comprises relatively thick walls and small fluid volume around the needle 19,
- a second area surrounds a middle portion of the needle 19 having relatively thin walls and/or walls with folding features e.g. portions with very thin wall thickness or in another way prepared to ease length reduction of the membrane 17, and
- a third area which area is provided with means for or adapted to attach the membrane 17 to the holding parts 61 e.g. by fitting around a protruding part 61a of the holding part 61.

Also a membrane 17 according to the present invention need not be abutted to or supported by surrounding walls of rigid material, this means that the membrane 17 is free to deform e.g. by bulking outwards in one or more bulk(s) depending on the folding features of the second area when pressure is put on the membrane 17. Without rigid material surrounding the sides of the membrane 17, also the sides of the membrane 17 - and not only the end - can also be penetrated by e.g. a syringe thereby adding fluid to the inside of the membrane 17 i.e. there is free access to at least part of the side area of the membrane 17.

The first area comprises the material of the membrane 17 which is positioned furthest away from the device i.e. the closed end piece which has to be penetrated by a needle 19 in order to form contact with e.g. a reservoir part placed outside the bubble shaped membrane 17. The membrane material in this first area is formed as a cavity having sides placed along the needle, a closed end to be penetrated by the needle 19, and an open end through which the needle 19 enters. The sides have a minimum material thickness b₂ which is large enough to make the first area non-deformable but the material thickness need not be constant all the way around the needle or in the whole length of the first area. The actual thickness or thickness distribution will depend on the choice of material and the dimensions e.g. length and diameter of the first area. Also the distance between the connector needle 19 and the membrane 17 will be of importance when determining how the membrane will be deformed when subjected to a pressure from the closed end i.e. if the distance between the outer surface of the connector needle 19 and the inner wall of the membrane is very small the walls of the membrane will when they are pushed back be guided by the contact with the connector needle 19 and not by the thickness i.e. the rigidity of the membrane material.

The second area comprises a portion of the membrane 17 which is positioned between the first and the third area. At least a part of the material of the membrane 17 in the second area has the thickness b₁, and the second area may have the form of one or more hollow truncated cone(s) or a cylinder(s) forming thick and thin parts in the membrane material in order to form folding features which define exactly where the membrane material will deform and how the material will deform. Normally the folding features are shaped to make the membrane material deform outward but as the rigid walls of the needle 19 assures that the fluid can keep flowing inside the needle 19, the membrane might as well deform inwards. Normally: b₂ > b₁ and for some materials often used for membranes of this type: b₂ ≥ 1.5·b₁. If the distance between the connector needle 19 and the membrane 17 in the first area is very small i.e. below 0.5 mm, the membrane thickness b2 might be smaller than b1 as the membrane can then wrinkle or fold when being pushed back along the connector needle 19. In this case the connector needle 19 placed inside the membrane 17 functions as a guide and a support for the membrane 17.

The third area comprises a part of the membrane material which is shaped in such a way that it can be used to fasten the membrane 17 to the holding part 61. The third area can comprise membrane material in a thickness and flexibility which e.g. makes it adequate for fitting over and squeezing around a protruding part 61a of the holding part 61 provided e.g. around the needle 19, or it can e.g. be shaped with an outward brim of material which makes it possible to squeeze the brim between two parts of the holding part 61.

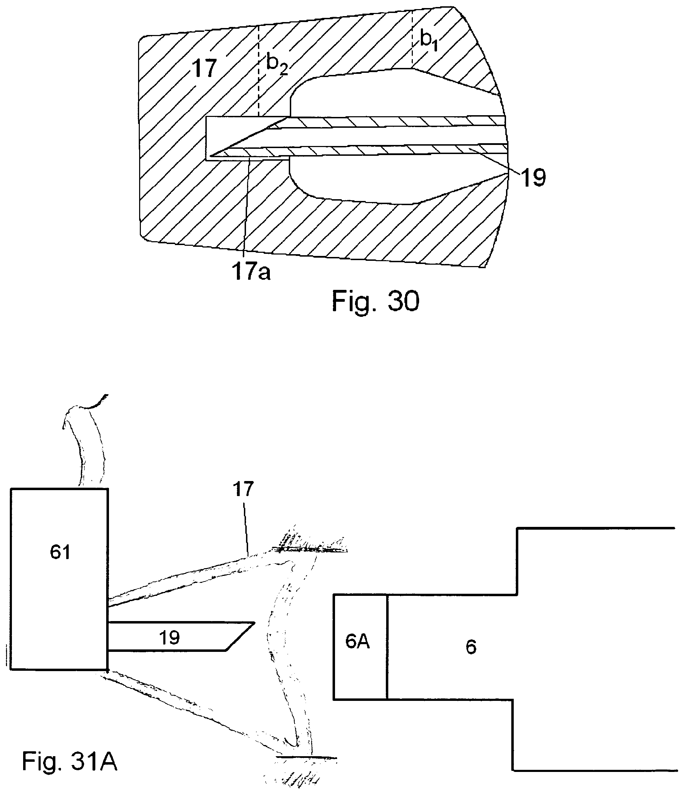

Fig. 30 shows an enlargement of a second embodiment of a membrane 17 to be used according to the invention. This bubble membrane 17 also completely surrounds the part of the connector needle 19 which protrudes from the surface of the not shown holding part 61 in which the connector needle 19 is embedded.

According to this embodiment the connector needle 19 is positioned so close to the membrane 17 that it might touch the bubble membrane 17 even when no pressure is put on the membrane 17 i.e. the passage 17a between the needle 19 and the membrane 17 represents a distance between membrane 17 and needle 19 close to 0, normally the distance will be less than 0.5 mm. When the membrane 17 is positioned this close to the connector needle 19, the needle 19 functions as guiding means when pressure is put on the membrane 17 which means that it will be easier to predict the folding of the membrane.

The needle 19 does not pierce the membrane 17 when no pressure is put on the membrane, this ensures filling of the membrane 17 with sterilizing gas such as Eto and distribution of the gas. The flexible membrane 17 should include a small vent e.g. a small cut in the side of the membrane 17 e.g. in the second area which cut opens when the membrane 17 is pushed over the needle 19. The vent allows for pressure relief.

Fig. 31A shows another embodiment of a bubble shaped membrane 17. According to this embodiment the reservoir 6 which is provided with an entrance protecting membrane 6A is pushed toward the bubble membrane 17 covering the connector needle 19. The bubble membrane 17 is made of a flexible material which makes it possible for the membrane to be deformed to such an extent that the connector needle 19 can penetrate the protecting membrane 6A and extend into the reservoir thereby providing access to the fluid reservoir 6.

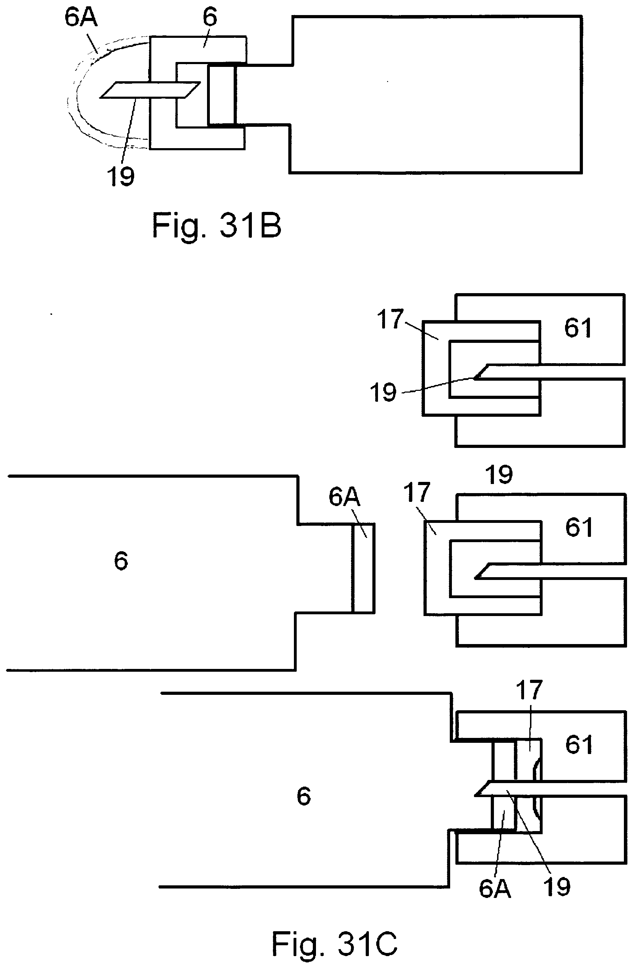

Fig. 31B shows another embodiment where a bubble membrane 6A is mounted at the outlet of a reservoir 6 which outlet can be connected to the fluid path of the connection path 3. The not shown end of the fluid path connecting to the reservoir 6 is provided with a membrane protecting the entrance of the fluid path during periods where the fluid path is not connected to the reservoir 6. According to this embodiment the fluid path need not be provided with a connector needle 19 as the connector needle 19 is part of the reservoir 6.

Fig. 31C shows yet another embodiment of a bubble membrane 17 and how the reservoir is pressed against the connector needle 19 in order to provide a fluid path for the medication contained in the reservoir 6. The bubble membrane 17 is flexible and is able to be reduced in size in such a way that it allows the entrance of the reservoir 6 to be pressed into the opening in the connection part 3 which surrounds the membrane 17 and the connector needle 19 i.e. the length of the membrane 17 can be reduced without the diameter of the membrane 17 being extended. According to the shown embodiment the material of the membrane will be folded inwards.

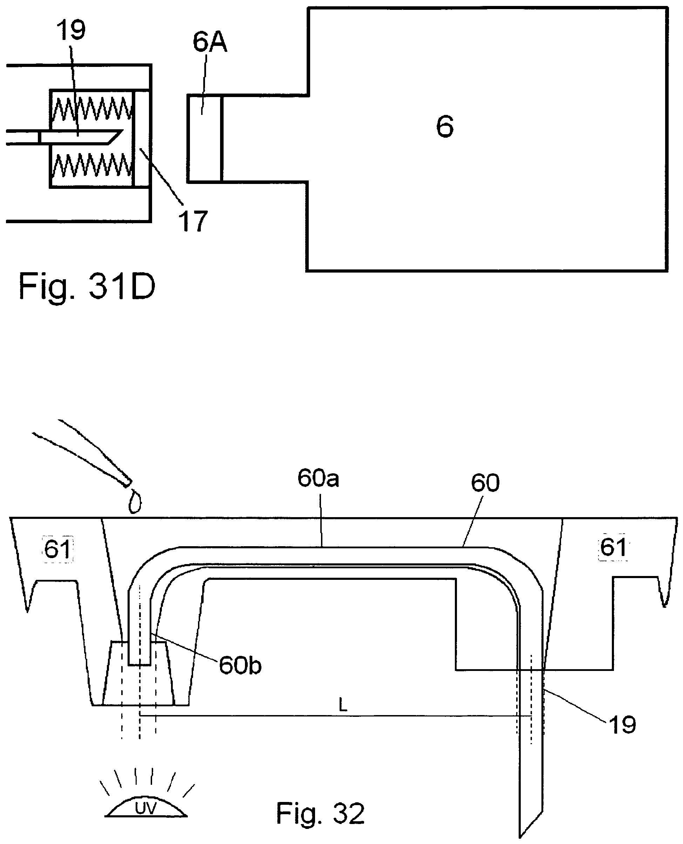

Fig. 31D shows yet another embodiment of a membrane 17 protecting the opening to the connection part 3. This membrane 17 is not bubble shaped but it provides a wall in a space surrounding the connector needle 19. The wall is pliant i.e. it will move back when the reservoir is pressed against it. The membrane wall 17 is kept in position by one or more springs i.e. the membrane 17 is able to return to the original position when the pressure from the reservoir 6 which keeps it in place is released. The opening in which the membrane slides back and forth closely fits the connecting part of the reservoir 6.

Fig. 32 discloses a fourth embodiment of a fluid connection according to the invention. This embodiment illustrates a method to uptake tolerances with regards to tolerances on the bended tube 60 which is also referred to as a needle. A bended tube 60 having two bends of each 90° as shown in fig. 32 will have a length tolerance between the bends, the tube 60 according to this embodiment is constructed of a connector needle 19, a blunt end needle 60b at the opposite end of the tube 60 and a connector piece 60a between the two 90° bends. The length of respectively the connector needle 19 and the blunt end needle 60b are supposed to fit into two through holes in the holding part 61. The two through holes have to have a tolerance allowing both the connector needle 19 and the blunt end needle 60b to enter the desired position in the holding parts 61. One way to ensure that the two ends will fit into the holes is to make the through holes large enough to obtain the tolerance of both the minimum and maximum material conditions. This though is not a good idea for several reasons: 1. if the tube 60 is to be glued into the connection part 3, the glue will run through the holes in too large amounts, 2) not enough control of the needle tip positions are obtained.

In order to get a tight control over the needle tip position interference fit on the connector needle 19 would be beneficial; interference fit would prevent the glue from running through the through hole and would make it possible to place the needle tip with great precision. Then all of the tolerance would have to be taken into account in the end of the blunt needle 60b and this can be done e.g. by making an elongated through hole for the blunt end needle which through hole in the dimension perpendicular to the length of the connector piece 60a is just larger than the outer diameter of the tube 60, and in the dimension parallel to the length of the connection piece 60a is long enough to take up all the tolerance i.e. this dimension could be e.g. 1½-2 times the diameter of the tube 60.

This however does not solve the problem with regard to the glue running through the hole at the blunt needle end, although having one hole sealed mechanically makes it easier to control the flow of glue out of the other.

The inner parts shown in fig. 32 show a solution to the problem of providing interference fit and tolerance at the same time. In this embodiment a first end of the tube 60 i.e. the end providing the pointy connection needle 19 is fitted closely into a through hole in the holding part 61. The second end of the tube 60 i.e. the blunt end, is fitted into a through hole with a tolerance gap surrounding the tube. The through hole providing the tolerance and surrounding the second end discharge into a space with an increased diameter / dimension, this means than when glue is pressed into the opening around the tube 60 from the open side of the holding part 61, the flow of glue will be slowed down when having passed the tolerance gap. Further, when the holding parts 61 are irradiated with e.g. UV light, the UV-light will cure any glue that comes through the tolerance gap.

## Claims

1. An assembly comprising an inserter device (10), a penetrating member (7) and a base part (100), where
- the base part (100) comprises a surface adapted to be attached to a skin surface, a position adapted to receive and/or attach to the penetrating member (7), and means (14) adapted to secure the base part to the inserter device (10),
- the penetrating member (7) comprises a part to be placed subcutaneously or intramuscularly, a body (24) which is in contact with the inserter device (10) during insertion and with the base part (100) during use, and
- the inserter device (10) comprises a cavity for receiving the penetrating member (7), means (45) for accelerating the penetrating member (7) and bringing the penetrating member (7) to the receiving position in the base part (100) and means for penetrating the skin of the patient,
**characterized in that** the length of the joined assembly (I ₜₒₜₐₗ) in a dimension horizontal to the skin of the patient when attached to the patients skin before use is larger than the length of the base part (100) (I₂) alone and the inserter device (10) is releasable from the base part (100) by applying a force to the inserter device (10) or a part of the inserter device (10) in a direction different from a direction of insertion of the penetrating member (7), wherein the inserter device comprises
- a moving part (38) comprising guiding means (39) which guiding means (39) restrict the movement of the penetrating member (50) and guide the penetrating member (50) from a first to a second position in a first direction, i.e. the direction of insertion, towards the injection site, and
- a stationary housing (30) comprising guiding means (32) which guiding means (32) restrict the movement of the moving part (38), and
- the penetrating member (50) comprises transformation means (52) corresponding to the guiding means (39) of the moving part (38).

2. An assembly according to claim 1, wherein the height of the joined assembly (hₜₒₜₐₗ) before use is smaller than the individual heights of the inserter device (10) (h₁) and the base part (10) (h₂) added together.

3. An assembly according to claim 1 or 2, wherein the guiding means (32) guide the moving part (38) in a second direction which is linear and different from the first direction i.e. the direction of insertion.

4. An assembly according to any of the preceding claims, wherein the base part and the penetrating member comprises corresponding means adapted to attach the penetrating member (50) to the base part (100) during use and after insertion.

5. An assembly according to any of the preceding claims, wherein the inserter (10) comprises inserter attachment means (14) locking the inserter (10) to the base part before and during insertion of the penetrating member (7) which inserter attachment means (14) can be unlocked so that the inserted can be removed from the base part after insertion of the penetrating member (7), the inserter attachment means (14, 14A, 14B, 14PR/R, 14PL/L) comprise at least one protruding part and at least one corresponding opening.

6. An assembly according to claim 5, wherein either the at least one protruding part or the at least one corresponding opening part is positioned on a surface of the base part and a corresponding part either comprising a protruding part or an opening is positioned on a surface of the inserter (10).

7. An assembly according to any preceding claim, **characterized in that** the release of the inserter (10) from the base part is at least partly provided by release of a force in direction towards the base part.

8. An assembly according to claim 7, **characterized in that** the released force is provided by a spring unit (45) exercising a force directed to a proximal surface of the inserter housing and a distal surface of the base part (100).

9. An assembly according to claim 1, **characterized in that** the assembly comprises a leaf spring (36), which at one end is fastened unreleasably to a part of the inserter device (10) and at the other end touches a distal surface of the base part (100) before the inserter is released from the base part.

10. An assembly according to any of the preceding claims, where the penetrating part (50) which comprises a cannula part (7) together with the base part (100) which base part (100) is provided with an internal fluid path and constitutes an infusion part provided with a cannula part during use, and
- the cannula part (7) comprises a body (24) formed by a hard material having an inner through going opening which through going opening is in fluid contact with a cannula (22) providing fluid contact with the patient, the body (24) of the cannula part (7) has an opening (20) corresponding to the inlet or outlet opening (12) of the internal fluid path resulting in fluid contact between the internal fluid path and the cannula part (7) and these two corresponding openings (12, 20) do, when they are positioned opposite each other, allow unrestricted flow
- the internal fluid path comprises at least one inlet and one outlet opening (12, 13) through which a fluid can enter and exit the fluid path, and
- a sealing (18) is positioned between the cannula part (7) and the inlet/outlet opening (12) of the fluid path when the cannula part (7) is in position for use in order to keep the fluid path to the cannula tight.

11. An assembly according to claim 10, where the sealing (18) is surrounding the inlet/outlet opening (12) and/or the distance d1 between a centre line c of the cannula part and a point on the outer surface of the cannula part positioned at or above the upper edge of the sealing (18) is larger than the distance d2 between the centre line c of the cannula part and a point on the outer surface of the cannula part positioned at or below the lower edge of the sealing (18).

12. An assembly according to claim 10 or 11, wherein the body (24) of the cannula part (7) is provided with a sealing (18) before use or the opening (12) of the fluid path is provided with a sealing (18) before use.

13. An assembly according to any of the claims 10-12, wherein the body (24) of the cannula part (7) has at least a second opening (21) to the inner through going opening.

14. An assembly according to claim 13, wherein the second opening (21) to the inner through going opening is covered by a self closing membrane which membrane can be penetrated by a blunt or pointy needle.

15. An assembly according to any of the claims 10-14, wherein a membrane (17) completely covers an opening (13) giving access to a space in the assembly which membrane (17) is made of an elastic material penetrable by a needle, the membrane (17) is fastened around the opening (13) and the membrane (17) protrudes from the opening (13) and forms an air filled volume in front of the opening (13) which air filled volume can be reduced in size when a pressure is put on the membrane (17) from the outside, the inner surfaces of the membrane (17) define a passage (17a) at the first closed end of the membrane (17) through which a needle (19) can pass and the second end of the membrane (17) is adapted to attach the membrane (17) to a holding part (61).

16. An assembly according to claim 15, wherein the walls of the membrane (17) have a thickness and shape of the chosen membrane material making it possible, to maintain the protruding shape in a use position without the outer surfaces of the membrane (17) being supported with walls of rigid material.

17. An assembly according to any of the preceding claims, where the assembly comprises a fluid connection (60) having at least a first and a second opening (13, 12), i.e. an inlet and an outlet, where the first opening (13) forms a fluid connection to a medication supply (6) or the like and the second opening (12) forms a fluid connection to an opening in the body (24) of a separate cannula part (7) and an at least partly sub- or transcutaneous positioned cannula (22).

18. An assembly according to claim 17, wherein the fluid connection is attached to a surface plate (1) and has the form of a tube (60) made of a rigid material; normally the fluid connection (60) is fastened to the surface plate (1) by a holding part (61).

19. An assembly according to claim 18, wherein a pointy end of the tube (60) forms a connector needle (19) being the inlet to a connector part (3) and when pushing a reservoir (6) towards the inlet the connector needle (19) penetrates a membrane (17) completely covering a first opening (13) of the connector part (3).

20. An assembly according to any of the claims 17-19, wherein the tube (60) consists of a single piece.

21. An assembly according to claim 20, wherein the tube (60) is bent in an angle > 0 degrees in at least one position or the tube (60) is bent in an angle > 0 degrees in at least two positions.

## Patentansprüche

1. Anordnung, umfassend eine Einsetzervorrichtung (10), ein Eindringelement (7) und ein Basisteil (100), wobei
- das Basisteil (100) eine Oberfläche, die angepasst ist, um an einer Hautoberfläche befestigt zu werden, eine Position, die angepasst ist, um das Eindringelement (7) aufzunehmen und/oder daran befestigt zu werden, und Mittel (14), die angepasst sind, das Basisteil an dem Eindringelement (10) zu sichern, umfasst,
- das Eindringelement (7) ein subkutan oder intramuskulär zu platzierendes Teil, einen Körper (24), der während der Einsetzung mit der Einsetzervorrichtung (10) und während der Verwendung mit dem Basisteil (100) in Kontakt steht, umfasst, und
- die Einsetzervorrichtung (10) einen Hohlraum zum Aufnehmen des Eindringelements (7), Mittel (45) zum Beschleunigen des Eindringelements (7) zum Bringen des Eindringelements (7) in die Aufnahmeposition in dem Basisteil (100) und Mittel zum Eindringen in die Haut des Patienten umfasst,
**dadurch gekennzeichnet, dass** die Länge der verbundenen Anordnung (lₜₒₜₐₗ) in einer Dimension horizontal zur Haut des Patienten, wenn vor Verwendung an der Haut des Patienten befestigt, größer ist als die Länge des Basisteils (100) (l₂) allein, und die Einsetzervorrichtung (10) durch Ausüben einer Kraft auf die Einsetzervorrichtung (10) oder einen Teil der Einsetzervorrichtung (10) in einer Richtung, die von einer Einsetzungsrichtung des Eindringelements (7) verschieden ist, von dem Basisteil (100) lösbar ist, wobei die Einsetzervorrichtung umfasst
- ein bewegliches Teil (38), umfassend Führungsmittel (39), wobei die Führungsmittel (39) die Bewegung des Eindringelements (50) einschränken und das Eindringelement (50) in eine erste Richtung, d. h. die Einsetzungsrichtung, aus einer ersten zu einer zweiten Position hin zu der Injektionsstelle führen, und
- ein stationäres Gehäuse (30), umfassend Führungsmittel (32), wobei die Führungsmittel (32) die Bewegung des beweglichen Teils (38) einschränken, und
- wobei das Eindringelement (50) dem Führungsmittel (39) des beweglichen Teils (38) entsprechende Transformierungsmittel (52) umfasst.

2. Anordnung nach Anspruch 1, wobei die Höhe der verbundenen Anordnung (hₜₒₜₐₗ) vor Verwendung kleiner ist als die zusammenaddierten individuellen Höhen der Einsetzervorrichtung (10) (h₁) und des Basisteils (10) (h₂).

3. Anordnung nach Anspruch 1 oder 2, wobei die Führungsmittel (32) das bewegliche Teil (38) in eine zweite Richtung führen, welche linear und von der ersten Richtung, d. h. der Einsetzungsrichtung, verschieden ist.

4. Anordnung nach einem der vorstehenden Ansprüche, wobei das Basisteil und das Eindringelement entsprechende Mittel umfassen, daran angepasst, um während der Verwendung und nach der Einsetzung das Eindringelement (50) an dem Basisteil (100) zu befestigen.

5. Anordnung nach einem der vorstehenden Ansprüche, wobei der Einsetzer (10) Einsetzerbefestigungsmittel (14) umfasst, die den Einsetzer (10) vor und während der Einsetzung des Eindringelements (7) mit dem Basisteil verriegeln, wobei Einsetzerbefestigungsmittel (14) entriegelt werden können, sodass der Einsetzer nach Einsetzung des Eindringelements (7) von dem Basisteil entfernt werden kann, wobei die Einsetzerbefestigungsmittel (14, 14A, 14B, 14PR/R, 14PL/L) mindestens ein hervorstehendes Teil und mindestens eine entsprechende Öffnung umfassen.

6. Anordnung nach Anspruch 5, wobei entweder das mindestens eine hervorstehende Teil oder das mindestens eine entsprechende Öffnungsteil auf einer Oberfläche des Basisteils positioniert ist und ein entsprechendes Teil entweder ein hervorstehendes Teil umfasst oder eine Öffnung auf einer Oberfläche des Einsetzers (10) positioniert ist.

7. Anordnung nach einem vorstehenden Anspruch, **dadurch gekennzeichnet, dass** das Lösen des Einsetzers (10) aus dem Basisteil mindestens teilweise durch Lösen einer Kraft in Richtung hin zu dem Basisteil bereitgestellt wird.

8. Anordnung nach Anspruch 7, **dadurch gekennzeichnet, dass** die freigesetzte Kraft durch eine Federeinheit (45) bereitgestellt wird, die eine zu einer proximalen Oberfläche des Einsetzergehäuses und einer distalen Oberfläche des Basisteils (100) gerichtete Kraft ausübt.

9. Anordnung nach Anspruch 1, **dadurch gekennzeichnet, dass** die Anordnung eine Blattfeder (36) umfasst, welche an einem Ende unlösbar an einem Teil der Einsetzervorrichtung (10) festgemacht ist und an dem anderen Ende eine distale Oberfläche des Basisteils (100) berührt, bevor der Einsetzer aus dem Basisteil gelöst wird.

10. Anordnung nach einem der vorstehenden Ansprüche, wobei das eindringende Teil (50), welches ein Kanülenteil (7) zusammen mit dem Basisteil (100) umfasst, welches Basisteil (100) mit einem internen Fluidweg bereitgestellt wird und einen während der Verwendung mit einem Kanülenteil bereitgestellten Infusionsteil ausmacht, und
- das Kanülenteil (7) einen aus einem harten Material mit einer inneren durchgehenden Öffnung gebildeten Körper (24) umfasst, wobei die innere durchgehende Öffnung in Fluidkontakt mit einer Kanüle (22) steht, die Fluidkontakt mit dem Patienten bereitstellt, wobei der Körper (24) des Kanülenteils (7) eine Öffnung (20) aufweist, die der Einlass- oder Auslassöffnung (12) des internen Fluidwegs entspricht, resultierend in Fluidkontakt zwischen dem internen Fluidweg und dem Kanülenteil (7), und wobei diese zwei entsprechenden Öffnungen (12, 20) uneingeschränkten Fluss erlauben, wenn sie einander gegenüberliegend positioniert sind
- der interne Fluidweg mindestens eine Einlass- und eine Auslassöffnung (12, 13) umfasst, durch welche ein Fluid in den Fluidweg einfließen und ihn verlassen kann, und
- eine Dichtung (18) zwischen dem Kanülenteil (7) und der Einlass-/Auslassöffnung (12) des Fluidwegs positioniert ist, wenn das Kanülenteil (7) in Position zur Verwendung ist, um den Fluidweg zur Kanüle dicht zu halten.

11. Anordnung nach Anspruch 10, wobei die Dichtung (18) die Einlass-/Auslassöffnung (12) umgibt und/oder der Abstand d1 zwischen einer Mittellinie c des Kanülenteils und einem an oder über dem oberen Rand der Dichtung (18) positionierten Punkt auf der Außenoberfläche des Kanülenteils größer ist als der Abstand d2 zwischen der Mittellinie c des Kanülenteils und einem an oder unter dem unteren Rand der Dichtung (18) positionierten Punkt auf der Außenoberfläche des Kanülenteils.

12. Anordnung nach Anspruch 10 oder 11, wobei der Körper (24) des Kanülenteils (7) vor Verwendung mit einer Dichtung (18) bereitgestellt ist oder die Öffnung (12) des Fluidwegs vor Verwendung mit einer Dichtung (18) bereitgestellt ist.

13. Anordnung nach einem der Ansprüche 10-12, wobei der Körper (24) des Kanülenteils (7) mindestens eine zweite Öffnung (21) zur inneren durchgehenden Öffnung aufweist.

14. Anordnung nach Anspruch 13, wobei die zweite Öffnung (21) zu der inneren durchgehenden Öffnung durch eine selbstschließende Membran bedeckt ist, wobei die Membran durch eine stumpfe oder spitze Nadel penetriert werden kann.

15. Anordnung nach einem der Ansprüche 10-14, wobei eine Membran (17) eine Öffnung (13), die Zugang zu einem Raum in dem Anordnung gibt, vollständig bedeckt, wobei die Membran (17) aus einem elastischen, durch eine Nadel penetrierbaren Material hergestellt ist, die Membran (17) um die Öffnung (13) herum festgemacht ist und die Membran (17) aus der Öffnung (13) hervorsteht und ein luftgefülltes Volumen vor der Öffnung (13) bildet, wobei das luftgefüllte Volumen in der Größe reduziert werden kann, wenn von außerhalb ein Druck an die Membran (17) angelegt wird, wobei die Innenoberflächen der Membran (17) an dem ersten geschlossenen Ende der Membran (17) einen Durchgang (17a) definieren, durch welchen eine Nadel (19) durchgehen kann, und das zweite Ende der Membran (17) daran angepasst ist, die Membran (17) an einem Halteteil (61) zu befestigen.

16. Anordnung nach Anspruch 15, wobei die Wände der Membran (17) mit einer Dicke und Form des gewählten Membranmaterials es ermöglichen, die hervorstehende Form in einer Verwendungsposition aufrecht zu halten, ohne dass die Außenoberflächen der Membran (17) mit Wänden aus steifem Material gestützt werden.

17. Anordnung nach einem der vorstehenden Ansprüche, wobei die Anordnung eine Fluidverbindung (60) mit mindestens einer ersten und zweiten Öffnung (13, 12), d. h. einen Einlass und einen Auslass, umfasst, wobei die erste Öffnung (13) eine Fluidverbindung zu einer Arzneizufuhr (6) oder dergleichen bildet und die zweite Öffnung (12) eine Fluidverbindung zu einer Öffnung in dem Körper (24) eines separaten Kanülenteils (7) und mindestens teilweise sub- oder transkutan positionierter Kanüle (22) bildet.

18. Anordnung nach Anspruch 17, wobei die Fluidverbindung an einer Oberflächenplatte (1) befestigt ist und die Form einer aus einem steifen Material hergestellten Röhre (60) aufweist; wobei die Fluidverbindung (60) normalerweise an der Oberflächenplatte (1) durch ein Halteteil (61) festgemacht ist.

19. Anordnung nach Anspruch 18, wobei ein spitzes Ende der Röhre (60) eine Verbindungsnadel (19) bildet, die der Einlass zu einem Verbindungsteil (3) ist und eine Membran (17) penetriert, die eine erste Öffnung (13) des Verbindungsteils (3) vollständig bedeckt, wenn ein Behälter (6) hin zu dem Einlass der Verbindungsnadel (19) gedrückt wird.

20. Anordnung nach einem der Ansprüche 17-19, wobei die Röhre (60) aus einem einzelnen Stück besteht.

21. Anordnung nach Anspruch 20, wobei die Röhre (60) in mindestens einer Position in einem Winkel > 0 Grad gebogen ist oder die Röhre (60) in mindestens zwei Positionen in einem Winkel > 0 Grad gebogen ist.

## Revendications

1. Ensemble comprenant un dispositif d'insertion (10), un élément de pénétration (7) et une partie de base (100), dans lequel :
- la partie de base (100) comprend une surface qui est à même d'être fixée à une surface de peau, une position qui est à même de recevoir l'élément de pénétration (7) et/ou de s'y fixer et des moyens (14) qui sont à même de fixer la partie de base au dispositif d'insertion (10),
- l'élément de pénétration (7) comprend une partie à placer en mode sous-cutané ou intramusculaire, un corps (24) qui est en contact avec le dispositif d'insertion (10) au cours de l'insertion et avec la partie de base (100) en cours d'utilisation et
- le dispositif d'insertion (10) comprend une cavité pour recevoir l'élément de pénétration (7), des moyens (45) pour accélérer l'élément de pénétration (7) et amener l'élément de pénétration (7) en position réceptrice dans la partie de base (100) et des moyens pour pénétrer dans la peau du patient,
**caractérisé en ce que** la longueur de l'ensemble joint (lₜₒₜₐₗ) dans une dimension horizontale par rapport à la peau du patient lorsqu'il est fixé à la peau du patient avant utilisation est plus grande que la longueur de la partie de base (100) (l₂) seule et le dispositif d'insertion (10) peut être libéré de la partie de base (100) en appliquant une force au dispositif d'insertion (10) ou à une partie du dispositif d'insertion (10) dans un sens différent d'un sens d'insertion de l'élément de pénétration (7), dans lequel le dispositif d'insertion comprend
- une partie mobile (38) comprenant des moyens de guidage (39), lesquels moyens de guidage (39) restreignent le mouvement de l'élément de pénétration (50) et guident l'élément de pénétration (50) d'une première dans une seconde position dans un premier sens, c'est-à-dire le sens d'insertion, vers le site d'injection et
- un boîtier fixe (30) comprenant des moyens de guidage (32), lesquels moyens de guidage (32) restreignent le mouvement de la partie mobile (38) et
- l'élément de pénétration (50) comprend des moyens de transformation (52) correspondant aux moyens de guidage (39) de la partie mobile (38).

2. Ensemble selon la revendication 1, dans lequel la hauteur de l'ensemble joint (hₜₒₜₐₗ) avant utilisation est plus petite que les hauteurs individuelles du dispositif d'insertion (10) (h₁) et de la partie de base (10) (h₂) ajoutées l'une à l'autre.

3. Ensemble selon la revendication 1 ou 2, dans lequel les moyens de guidage (32) guident la partie mobile (38) dans un second sens qui est linéaire et différent du premier sens, c'est-à-dire du sens d'insertion.

4. Ensemble selon l'une quelconque des revendications précédentes, dans lequel la partie de base et l'élément de pénétration comprennent des moyens correspondants qui sont à même de fixer l'élément de pénétration (50) à la partie de base (100) en cours d'utilisation et après insertion.

5. Ensemble selon l'une quelconque des revendications précédentes, dans lequel le dispositif d'insertion (10) comprend des moyens (14) de fixation du dispositif d'insertion verrouillant le dispositif d'insertion (10) à la partie de base avant et pendant l'insertion de l'élément de pénétration (7), lesquels moyens (14) de fixation du dispositif d'insertion peuvent être déverrouillés de sorte que le dispositif d'insertion puisse être retiré de la partie de base après insertion de l'élément de pénétration (7), les moyens (14, 14A, 14B, 14PR/R, 14PL/L) de fixation du dispositif d'insertion comprennent au moins une partie saillante et au moins une ouverture correspondante.

6. Ensemble selon la revendication 5, dans lequel l'une ou l'autre de la au moins une partie saillante et de la au moins une partie d'ouverture correspondante est positionnée sur une surface de la partie de base et une partie correspondante comprenant une partie saillante ou une ouverture est positionnée sur une surface du dispositif d'insertion (10).

7. Ensemble selon l'une quelconque des revendications précédentes, **caractérisé en ce que** la libération du dispositif d'insertion (10) de la partie de base est au moins en partie assurée par libération d'une force dans le sens de la partie de base.

8. Ensemble selon la revendication 7, **caractérisé en ce que** la force libérée est fournie par une unité de ressort (45) exerçant une force dirigée sur une surface proximale du boîtier du dispositif d'insertion et une surface distale de la partie de base (100).

9. Ensemble selon la revendication 1, **caractérisé en ce que** l'ensemble comprend un ressort à lames (36) qui, à une extrémité, est fixé de manière non libérable à une partie du dispositif d'insertion (10) et, à l'autre extrémité, touche une surface distale de la partie de base (100) avant que le dispositif d'insertion ne soit libéré de la partie de base.

10. Ensemble selon l'une quelconque des revendications précédentes, dans lequel la partie de pénétration (50) qui comprend une partie de canule (7) conjointement avec la partie de base (100), laquelle partie de base (100) est pourvue d'un trajet de fluide interne et constitue une partie d'infusion pourvue d'une partie de canule en cours d'utilisation et
- la partie de canule (7) comprend un corps (24) formé par un matériau dur ayant une ouverture traversante interne, laquelle ouverture traversante étant en contact fluidique avec une canule (22) fournissant un contact fluidique avec le patient, le corps (24) de la partie de canule (7) a une ouverture (20) correspondant à l'ouverture d'entrée ou de sortie (12) du trajet de fluide interne, ce qui entraîne un contact fluidique entre le trajet de fluide interne et la partie de canule (7) et ces deux ouvertures correspondantes (12, 20) permettent, lorsqu'elles sont disposées à l'opposé l'une de l'autre, un écoulement non restreint,
- le trajet de fluide interne comprend au moins une ouverture d'entrée et une ouverture de sortie (12, 13) à travers lesquelles un fluide peut pénétrer dans le trajet de fluide et en sortir, et
- un joint d'étanchéité (18) est appliqué entre la partie de canule (7) et l'ouverture d'entrée/sortie (12) du trajet de fluide lorsque la partie de canule (7) est dans une position pour une utilisation afin de maintenir étanche le trajet de fluide vers la canule.

11. Ensemble selon la revendication 10, dans lequel le joint d'étanchéité (18) entoure l'ouverture d'entrée/sortie (12) et/ou la distance d1 entre une ligne centrale c de la partie de canule et un point sur la surface externe de la partie de canule positionné dans ou sur le bord supérieur du joint d'étanchéité (18) est plus grande que la distance d2 entre la ligne centrale c de la partie de canule et un point sur la surface externe de la partie de canule positionné dans ou sous le bord inférieur du joint d'étanchéité (18).

12. Ensemble selon la revendication 10 ou 11, dans lequel le corps (24) de la partie de canule (7) est pourvu d'un joint d'étanchéité (18) avant utilisation ou l'ouverture (12) du trajet de fluide est pourvue d'un joint d'étanchéité (18) avant utilisation.

13. Ensemble selon l'une quelconque des revendications 10 à 12, dans lequel le corps (24) de la partie de canule (7) a au moins une seconde ouverture (21) menant à l'ouverture traversante interne.

14. Ensemble selon la revendication 13, dans lequel la seconde ouverture (21) donnant sur l'ouverture traversante interne est recouverte par une membrane de fermeture automatique, laquelle membrane peut être pénétrée par une aiguille émoussée ou pointue.

15. Ensemble selon l'une quelconque des revendications 10 à 14, dans lequel une membrane (17) recouvre complètement une ouverture (13) donnant accès à un espace dans l'ensemble, laquelle membrane (17) est constituée d'un matériau élastique pénétrable par une aiguille, la membrane (17) est fixée autour de l'ouverture (13) et la membrane (17) fait saillie de l'ouverture (13) et forme un volume rempli d'air devant l'ouverture (13), lequel volume rempli d'air peut être réduit en taille lorsqu'une pression est appliquée sur la membrane (17) de l'extérieur, les surfaces internes de la membrane (17) définissent un passage (17a) à la première extrémité fermée de la membrane (17) à travers laquelle une aiguille (19) peut passer et la seconde extrémité de la membrane (17) est à même de fixer la membrane (17) à une partie de maintien (61).

16. Ensemble selon la revendication 15, dans lequel les parois de la membrane (17) ont une épaisseur et une forme du matériau de membrane choisi qui permet de maintenir la forme saillante en position d'utilisation sans les surfaces externes de la membrane (17) qui sont supportées par des parois de matériau rigide.

17. Ensemble selon l'une quelconque des revendications précédentes, dans lequel l'ensemble comprend un raccord fluidique (60) ayant au moins une première et une seconde ouverture (13, 12), c'est-à-dire une entrée et une sortie, où la première ouverture (13) forme un raccord fluidique avec une alimentation de médicament (6) ou analogue et la seconde ouverture (12) forme un raccord fluidique avec une ouverture dans le corps (24) d'une partie de canule séparée (7) et une canule positionnée au moins en partie en mode sous-cutané ou transcutané (22).

18. Ensemble selon la revendication 17, dans lequel le raccord fluidique est fixé à une plaque de surface (1) et a la forme d'un tube (60) constitué d'un matériau rigide ; normalement le raccord fluidique (60) est fixé à la plaque de surface (1) par une partie de maintien (61).

19. Ensemble selon la revendication 18, dans lequel une extrémité pointue du tube (60) forme une aiguille de raccord (19) qui est l'entrée dans une partie de raccordement (3) et, lorsque l'on pousse un réservoir (6) vers l'entrée, l'aiguille de raccordement (19) pénètre dans une membrane (17) en recouvrant complètement une première ouverture (13) de la partie de raccordement (3).

20. Ensemble selon l'une quelconque des revendications 17 à 19, dans lequel le tube (60) est constitué d'une seule pièce.

21. Ensemble selon la revendication 20, dans lequel le tube (60) est cintré sous un angle > 0 degré dans au moins une position ou le tube (60) est cintré sous un angle > 0 degré dans au moins deux positions.
